# EUROPEAN PATENT SPECIFICATION

(11) **EP 2 828 405 B1**
(45) Date of publication and mention of the grant of the patent: **28.06.2017**
(21) Application number: 13764207.0
(22) Date of filing: 20.03.2013
(51) Int. Cl.: C12Q 1/68

(54) **MARKER PANEL FOR DETECTING CANCER**
MARKERPANEL FÜR DEN NACHWEIS VON KREBS
PANEL DE MARQUEURS POUR LA DÉTECTION DU CANCER

(30) Priority: 20.03.2012 US 201261613252 P
(43) Date of publication of application: 28.01.2015
(73) Proprietor: Exact Sciences Corporation, Madison, Wisconsin 53719 (US); MAYO FOUNDATION FOR MEDICAL EDUCATION AND RESEARCH, Rochester, MN 55905 (US)
(72) Inventor: WEISBURG, William G., San Diego California 92128 (US); LIDGARD, Graham P., Madison Wisconsin 53717 (US); AHLQUIST, David A., Rochester Minnesota 55902 (US); TAYLOR, William R., Lake City Minnesota 55041 (US)
(74) Representative: Glawe, Delfs, Moll
(86) International application number: PCT/US2013/033047
(87) International publication number: WO 2013/142545

(56) References cited:
- EP-A2- 1 721 992
- WO-A1-2008/009478
- WO-A2-2005/017207
- US-A1- 2008 145 852
- US-A1- 2011 236 916
- H. ZOU ET AL: "Quantification of Methylated Markers with a Multiplex Methylation-Specific Technology", CLINICAL CHEMISTRY, vol. 58, no. 2, 22 December 2011 (2011-12-22), pages 375-383, XP055105588, ISSN: 0009-9147, DOI: 10.1373/clinchem.2011.171264
- AHLQUIST ET AL.: 'The Stool DNA Test ls More Accurate Than the Plasma Septin 9 Test in Detecting Colorectal Neoplasia.' CLINICAL GASTROENTEROLOGY AND HEPATOLOGY. vol. 10, no. 3, 20 October 2011, pages 272 - 7.E1., XP055167696
- WARREN ET AL.: 'Septin 9 methylated DNA is a sensitive and specific blood test for colorectal cancer.' BMC MEDICINE vol. 9, no. 133, 2011, pages 1 - 9., XP021131493
- ZOU ET AL.: 'Quantification of Methylated Markers with a Multiplex Methylation-Specific Technology.' CLIN CHEM. vol. 58, no. 2, 22 December 2011, pages 375 - 83, XP055105588

### SEQUENCE LISTING

<110> Exact Sciences Corporation
   Mayo Foundation for Medical Education and Research
   Weisburg, William G.
   Lidgard, Graham P.
   Ahlquist, David A.
   Taylor, William R.
<120> MARKER PANEL FOR DETECTING CANCER
<130> EXCT-32376/WO-1/ORD
<150> US 61/613,252
   <151> 2012-03-20
<160> 40
<170> PatentIn version 3.5
<210> 1
   <211> 41
   <212> DNA
   <213> Artificial sequence
<220>
   <223> Synthetic
<400> 1
   ctgtaggtgc gggtggacgt agtcacgtag ctccggctgg a 41
<210> 2
   <211> 48
   <212> DNA
   <213> Artificial sequence
<220>
   <223> Synthetic
<400> 2
   tccctcgcgc gtggcttccg ccttctgcgc ggctggggtg cccggtgg 48
<210> 3
   <211> 26
   <212> DNA
   <213> Artificial sequence
<220>
   <223> Synthetic
<400> 3
   gcgggacact ccgaaggcgc aaggag 26
<210> 4
   <211> 26
   <212> DNA
   <213> Artificial sequence
<220>
   <223> Synthetic
<400> 4
   cgcctggagc agaaagccgc gcacct 26
<210> 5
   <211> 34
   <212> DNA
   <213> Artificial sequence
<220>
   <223> Synthetic
<400> 5
   ccttgtcaca cgagccagtg ttagtaccta cacc 34
<210> 6
   <211> 44
   <212> DNA
   <213> Artificial sequence
<220>
   <223> Synthetic
<400> 6
   ggcctgctga aaatgactga atataaactt gtggtagttg gage 44
<210> 7
   <211> 46
   <212> DNA
   <213> Artificial sequence
<220>
   <223> Synthetic
<400> 7
   ctctattgtt ggatcatatt cgtccacaaa atgattctga attagc 46
<210> 8
   <211> 16
   <212> DNA
   <213> Artificial sequence
<220>
   <223> Synthetic
<400> 8
   ggcggttcgg gtatcg 16
<210> 9
   <211> 21
   <212> DNA
   <213> Artificial sequence
<220>
   <223> Synthetic
<400> 9
   cgtaatcacg taactccgac t 21
<210> 10
   <211> 21
   <212> DNA
   <213> Artificial sequence
<220>
   <223> Synthetic
<400> 10
   gacgcggagg cgagtcggtc g 21
<210> 11
   <211> 21
   <212> DNA
   <213> Artificial sequence
<220>
   <223> Synthetic
<400> 11
   cggttttcgt tcgttttttc g 21
<210> 12
   <211> 20
   <212> DNA
   <213> Artificial sequence
<220>
   <223> Synthetic
<400> 12
   gtaacttccg ccttctacgc 20
<210> 13
   <211> 20
   <212> DNA
   <213> Artificial sequence
<220>
   <223> Synthetic
<400> 13
   cgccgagggt tcgtttatcg 20
<210> 14
   <211> 21
   <212> DNA
   <213> Artificial sequence
<220>
   <223> Synthetic
<400> 14
   gtttaatttt cggtttcgtc g 21
<210> 15
   <211> 17
   <212> DNA
   <213> Artificial sequence
<220>
   <223> Synthetic
<400> 15
   ctcccgacgt cgctacg 17
<210> 16
   <211> 20
   <212> DNA
   <213> Artificial sequence
<220>
   <223> Synthetic
<400> 16
   cgccgaggcg gttttttgcg 20
<210> 17
   <211> 18
   <212> DNA
   <213> Artificial sequence
<220>
   <223> Synthetic
<400> 17
   tcgttgggta aggcgttc 18
<210> 18
   <211> 18
   <212> DNA
   <213> Artificial sequence
<220>
   <223> Synthetic
<400> 18
   aaacgaacac ccgaaccg 18
<210> 19
   <211> 22
   <212> DNA
   <213> Artificial sequence
<220>
   <223> Synthetic
<400> 19
   gacgcggagg cggttttttg tt 22
<210> 20
   <211> 15
   <212> DNA
   <213> Artificial sequence
<220>
   <223> Synthetic
<400> 20
   gcgggaggag gtgcc 15
<210> 21
   <211> 27
   <212> DNA
   <213> Artificial sequence
<220>
   <223> Synthetic
<400> 21
   tttgtttttt tgattaggtg tttaaga 27
<210> 22
   <211> 21
   <212> DNA
   <213> Artificial sequence
<220>
   <223> Synthetic
<400> 22
   caccaacctc ataaccttat c 21
<210> 23
   <211> 21
   <212> DNA
   <213> Artificial sequence
<220>
   <223> Synthetic
<400> 23
   ccacggacga tagtgttgtg g 21
<210> 24
   <211> 20
   <212> DNA
   <213> Artificial sequence
<220>
   <223> Synthetic
<400> 24
   aggcctgctg aaaatgactg 20
<210> 25
   <211> 22
   <212> DNA
   <213> Artificial sequence
<220>
   <223> Synthetic
<400> 25
   ctattgttgg atcatattcg tc 22
<210> 26
   <211> 19
   <212> DNA
   <213> Artificial sequence
<220>
   <223> Synthetic
<400> 26
   cttgtggtag ttggagcaa 19
<210> 27
   <211> 20
   <212> DNA
   <213> Artificial sequence
<220>
   <223> Synthetic
<400> 27
   gcgcgtccag tggcgtaggc 20
<210> 28
   <211> 22
   <212> DNA
   <213> Artificial sequence
<220>
   <223> Synthetic
<400> 28
   aaacttgtgg tagttggacc tt 22
<210> 29
   <211> 20
   <212> DNA
   <213> Artificial sequence
<220>
   <223> Synthetic
<400> 29
   gcgcgtcctg tggcgtaggc 20
<210> 30
   <211> 25
   <212> DNA
   <213> Artificial sequence
<220>
   <223> Synthetic
<400> 30
   tataaacttg tggtagttgg acctc 25
<210> 31
   <211> 20
   <212> DNA
   <213> Artificial sequence
<220>
   <223> Synthetic
<400> 31
   gcgcgtcccg tggcgtaggc 20
<210> 32
   <211> 21
   <212> DNA
   <213> Artificial sequence
<220>
   <223> Synthetic
<400> 32
   acttgtggta gttggagctc a 21
<210> 33
   <211> 20
   <212> DNA
   <213> Artificial sequence
<220>
   <223> Synthetic
<400> 33
   gcgcgtccat ggcgtaggca 20
<210> 34
   <211> 21
   <212> DNA
   <213> Artificial sequence
<220>
   <223> Synthetic
<400> 34
   acttgtggta gttggagctc t 21
<210> 35
   <211> 20
   <212> DNA
   <213> Artificial sequence
<220>
   <223> Synthetic
<400> 35
   gcgcgtcctt ggcgtaggca 20
<210> 36
   <211> 22
   <212> DNA
   <213> Artificial sequence
<220>
   <223> Synthetic
<400> 36
   aacttgtggt agttggagat gc 22
<210> 37
   <211> 20
   <212> DNA
   <213> Artificial sequence
<220>
   <223> Synthetic
<400> 37
   gcgcgtccct ggcgtaggca 20
<210> 38
   <211> 18
   <212> DNA
   <213> Artificial sequence
<220>
   <223> Synthetic
<400> 38
   ggtagttgga gctggtca 18
<210> 39
   <211> 20
   <212> DNA
   <213> Artificial sequence
<220>
   <223> Synthetic
<400> 39
   gcgcgtccac gtaggcaaga 20
<210> 40
   <211> 22
   <212> DNA
   <213> Artificial sequence
<220>
   <223> Synthetic
<400> 40
   ctattgttgg atcatattcg tc 22

## Description

### FIELD OF INVENTION

Provided herein is technology relating to detecting neoplasia and particularly, but not exclusively, to methods for detecting colorectal neoplasia by evaluating multiple gene markers in blood or plasma and stool.

### BACKGROUND

Colorectal cancer (CRC) remains the number two cancer in terms of mortality in the United States. Worldwide, CRC is increasingly common, now accounting for more than 600,000 deaths annually. Broadly applied preventive and early detection measures are critically needed to lessen this toll. To reduce incidence and mortality by screening, an interventional tool should maximize detecting both advanced precursor lesions and curable-stage cancers from throughout the colorectum and be patient friendly, available, and affordable. Conventional screening approaches do not achieve all these desired attributes. For example, proximal colon neoplasms are particularly underdetected by conventional approaches, including fecal blood tests, sigmoidoscopy, and colonoscopy as currently practiced. Because of poor precursor lesion detection rates, fecal blood tests have had minimal impact on CRC incidence. Accordingly, alternate or complementary strategies are needed to improve CRC screening performance, especially for detection of proximal advanced neoplasms.

Molecular detection of CRC offers the patient-friendly appeal of noninvasiveness. A host of compounds from the basic marker classes (e.g., protein, RNA, and DNA) have been assayed from blood, stool, and urine with varying results (see, e.g., Ahlquist DA. "Molecular detection of colorectal neoplasia", Gastroenterology 2010; 138: 2127-39; Tao S, et al. "Sensitivity estimates of blood-based tests for colorectal cancer detection: impact of overrepresentation of advanced stage disease", Am J Gastroenterol 2011; 106: 242-53). Neoplasm detection rates depend on the nature of the marker(s), the mechanism of marker release into the target medium, and marker levels within that medium. For the most accurate, precise, and sensitive detection of cancers, it is desirable that molecular markers discriminate colorectal neoplasia from normal colon at the tissue level, be continuously released into the target medium from both CRC and advanced precancers, and be present at concentrations sufficient for assay detection.

### SUMMARY OF THE INVENTION

Two emergent molecular approaches for the screening of colorectal neoplasia (e.g., CRC) are the plasma assay of methylated *Septin 9* (SEPT9) and a prototype next generation stool DNA (sDNA) test (Ahlquist DA. "The Stool DNA Test is More Accurate than the Plasma Septin 9 Test in Detecting Colorectal Neoplasia", Clinical Gastroenterology and Hepatology 2011). Herein is provided technology related to evaluating a combination of blood and stool DNA markers to provide an improved (e.g., more sensitive and more specific) detection of colorectal neoplasms..

Accordingly, provided herein is technology related to a method of screening for a colorectal neoplasm in a subject, the method comprising providing paired samples from a subject, wherein the paired samples comprise i) a stool sample, and ii) a blood sample or a plasma sample; assaying the stool sample to create a first data set, wherein the assaying comprises measuring the methylation state of at least one DNA marker; assaying the blood or a plasma sample to create a second data set, wherein the assaying comprises measuring the methylation state of at least one DNA marker; combining the first data set and the second data set to form a paired data set; and analyzing the paired data set to determine the presence or absence of a methylation profile indicative of colorectal neoplasm in the subject. In certain embodiments, the subject is human.

In some embodiments, assaying the stool sample comprises measuring the methylation states of a plurality of DNA markers. In certain preferred embodiments, assaying the stool sample comprises measuring the methylation states of at least three DNA markers, which in particularly preferred embodiments, assaying the stool sample comprises measuring the methylation states of at least four DNA markers. In certain embodiments, the marker is selected from the group consisting of a *vimentin* gene, an *NDRG4* gene, a *BMP3* gene, and a *TFPI2* gene, while in certain preferred embodiments, assaying the stool sample comprises measuring the methylation states of a *vimentin* gene, an *NDRG4* gene, a *BMP3* gene, and a *TFPI2* gene.

In certain embodiments, assaying the plasma sample comprises measuring the methylation state of at least one DNA marker not assayed in said stool sample. In preferred embodiments, the assaying of the plasma sample comprises measuring the methylation state of a *Septin 9* gene.

In some embodiments, additional indicators of colorectal health are determined. For example, in some embodiments, the screening method further comprises assaying the stool sample to detect and/or measure a mutant *KRAS* gene, (e.g., a mutant encoding a polypeptide comprising a substitution of the glycine at position 12 to serine, cysteine, arginine, aspartic acid, valine, or alanine, or of the gliycine at position 13 to aspartic acid). Other biological tests are performed in some embodiments, e.g., in some embodiments the methods comprise measuring a level of a fecal hemoglobin. The information from the multiple tests provides a dataset from which conclusions can be made regarding the state of the subject, e.g., to assess the health of the subject.

The DNA assays are not limited to any particular method, and many DNA assay methods find application in the present methods. In some preferred embodiments, the assaying the stool sample and/or the blood or plasma sample comprises the use of an amplification method such as a polymerase chain reaction, or a signal amplification assay, such as an invasive cleavage assay. In certain particularly preferred embodiments, the assaying comprises the use of a quantitative, allele-specific, real-time target and signal amplification (QuARTS) assay. In addition, the technology is not limited in the methods used to prepare the samples and provide a DNA for testing. For example, in some embodiments, a DNA is isolated from the stool sample or from the blood or from the plasma sample using direct gene capture, e.g., as detailed in U.S. Pat. Appl. Ser. No. 61/485386 or by a related method.

In some embodiments, DNA is isolated from the stool sample and/or from the blood or plasma sample using direct gene capture.

In some embodiments, the screening method is analyzed to detect a neoplasm that is cancer, while in some embodiments, the neoplasm is an adenoma. In some embodiments, the neoplasm is pre-cancerous.

In certain embodiments, the present invention provides a method of screening for a colorectal neoplasm in a subject, the method comprising:
a) providing paired samples from a subject, wherein said paired samples comprise
   i) a stool sample, and
   ii) a blood sample or a plasma sample;
b) isolating a first DNA from said stool sample;
c) isolating a second DNA from said stool sample;
d) isolating a third DNA from said blood sample or from said plasma sample;
e) measuring a first methylation state of a first gene using the first DNA;
f) measuring a second methylation state of a second gene using the second DNA;
g) measuring a third methylation state of a third gene using the third DNA.
h) combining data from said measuring of said first methylation state, said second methylation state and said third methylation state to form a paired data set;
g) analyzing said paired data set to determine the presence or absence of a methylation profile indicative of colorectal neoplasm in said subject.
In some embodiments, the first and the second gene are selected from the group consisting of *vimentin* gene, an *NDRG4* gene, a *BMP3* gene, and a *TFPI2* gene. In certain preferred embodiments, the third gene is a *Septin 9* gene

The isolation of the DNA from the paired samples is not limited to any particular method. In some embodiments, DNA is isolated by a hybrid capture method. In certain preferred embodiments, the isolation of the first DNA from the stool sample and the isolation of the second DNA from the stool sample are performed sequentially. In certain preferred embodiments, additional DNAs are further sequentially isolated from the stool sample.

It is contemplated that there are no limitations on the types of subjects for which the methods are appropriate. In specific embodiments, the subject is a human.

Additional embodiments will be apparent to persons skilled in the relevant art based on the teachings contained herein.

### BRIEF DESCRIPTION OF THE DRAWINGS

These and other features, aspects, and advantages of the present technology will become better understood with regard to the following drawings:
Figure 1 provides a graph comparing detection rates of large colorectal adenomas by stool DNA and plasma *septin 9* tests (n = 22). Median adenoma size was 2.0 cm (range 1.0 - 5.4), and 55% were proximal to the splenic flexure.

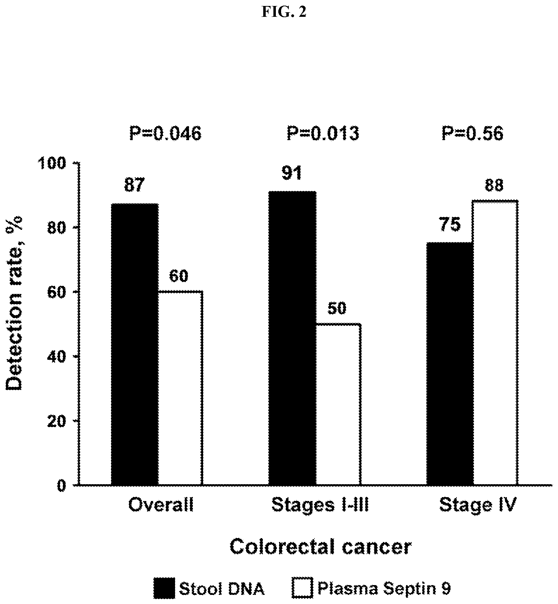

Figure 2 provides a graph comparing detection rates of colorectal cancer by stool DNA and plasma *septin 9* testing. Rates are compared for the overall group, n = 30; the subset without distant metastases (stages I-III), n = 22; and the subset with distant metastases (stage IV), n = 8.

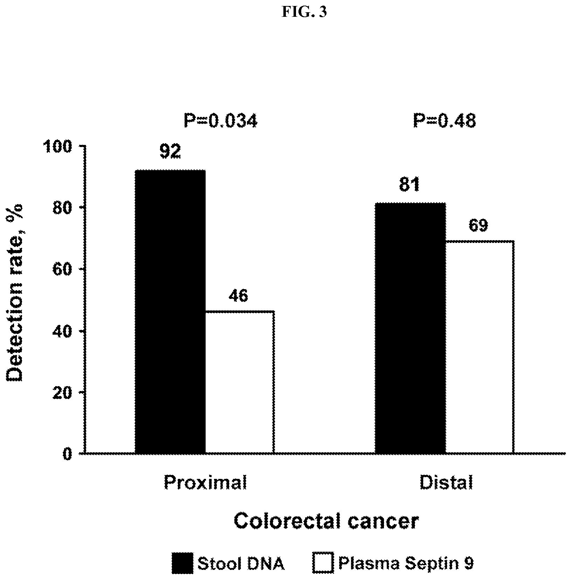

Figure 3 provides a graph comparing colorectal cancer detection rates by stool DNA and plasma *septin 9* testing according to tumor site. See footnote in Table 1 for definitions of proximal and distal.

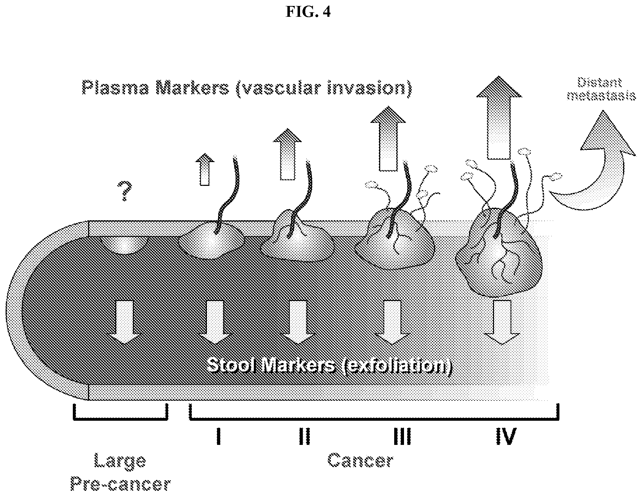

Figure 4 provides a schematic diagram comparing molecular marker release from colorectal neoplasms into target media. This conceptual model shows proportional differences (illustrated by arrow sizes) expected in rates of marker release into the bloodstream via the mechanism of vascular invasion and into the the stool via the mechanism of exfoliation during progressive phases of tumorigenesis. Marker release into the bloodstream from precursor lesions is negligible but increases progressively with advancing stages of cancer. In contrast, marker release by exfoliation into stool occurs at comparable rates from large precancers and all stages of cancer.

### DETAILED DESCRIPTION OF THE INVENTION

Embodiments of the invention are described in this summary, and in the Summary of the Invention, above, which is incorporated here by reference. Although the invention has been described in connection with specific embodiments, it should be understood that the invention as claimed should not be unduly limited to such specific embodiments.

Provided herein is technology relating to detecting cancer and particularly, but not exclusively, to methods for detecting colorectal neoplasia by evaluating multiple markers in paired samples from a subject (e.g., a patient) wherein the paired sample comprises a blood or plasma sample and a stool sample. In particular, the technology relates to screening by assaying plasma or blood for methylated *Septin 9* (SEPT9), assaying stool DNA for at least one marker, *e.g.,* a methylation marker, preferably a plurality of methylated markers, more preferably comprising one or more of *vimentin, NDRG4, BMP3,* and *TFPI2.*

### DEFINITIONS

To facilitate an understanding of the present technology, a number of terms and phrases are defined below. Additional definitions are set forth throughout the detailed description.

Throughout the specification and claims, the following terms take the meanings explicitly associated herein, unless the context clearly dictates otherwise. The phrase "in one embodiment" as used herein does not necessarily refer to the same embodiment, though it may. Furthermore, the phrase "in another embodiment" as used herein does not necessarily refer to a different embodiment, although it may.

In addition, as used herein, the term "or" is an inclusive "or" operator and is equivalent to the term "and/or" unless the context clearly dictates otherwise. The term "based on" is not exclusive and allows for being based on additional factors not described, unless the context clearly dictates otherwise. In addition, throughout the specification, the meaning of "a", "an", and "the" include plural references. The meaning of "in" includes "in" and "on."

As used herein, the term "neoplasm" refers to "an abnormal mass of tissue, the growth of which exceeds and is uncoordinated with that of the normal tissues" See, e.g., Willis RA, "The Spread of Tumors in the Human Body", London, Butterworth & Co, 1952.

As used herein, the term "paired samples" refers to the pairing of a blood-based sample (e.g., whole blood, plasma) with a stool sample collected from the same subject or patient. Paired samples may be collected contemporaneously, e.g., during a single medical appointment or visit, or paired samples may be collected at different times and places, *e.g.,* wherein blood drawn during medical appointment is paired with a stool sample collected at home by the subject, or during a different medical appointment. Paired samples need not be collected on the same day, but may be collected within any timeframe during which the colorectal health status of the subject would reasonably be expected to be sufficiently unchanged that particular assay result would not substantially change between the beginning of the time period and the end of the time period, *e.g.,* a day, a week, a month, *etc.*

As used herein, "methylation" refers to cytosine methylation at positions C5 or N4 of cytosine, the N6 position of adenine, or other types of nucleic acid methylation. *In vitro* amplified DNA is unmethylated because *in vitro* DNA amplification methods do not retain the methylation pattern of the amplification template. However, "unmethylated DNA" or "methylated DNA" can also refer to amplified DNA whose original template was unmethylated or methylated, respectively.

A "methylation profile" refers to a set of data representing the methylation states of one or more genes or loci (*e.g.,* a "panel") from, for example, the genome of a subject, or cells or tissues from a subject. The profile can indicate the methylation state of every base in an individual, or can comprise information regarding a subset of the base pairs (e.g., the methylation state of specific genes or markers) in a genome, or can comprise information regarding regional methylation density of each locus.

"Methylation status" refers to the presence, absence, and/or quantity of methylation at a particular nucleotide or nucleotides within a portion of DNA. The methylation status of a particular DNA sequence (e.g., a gene marker or DNA region as described herein) can indicate the methylation state of every base in the sequence or can indicate the methylation state of a subset of the base pairs (e.g., of one or more cytosines) within the sequence, or can indicate information regarding regional methylation density within the sequence without providing precise information of where in the sequence the methylation occurs. The methylation status can optionally be represented or indicated by a "methylation value." A methylation value can be generated, for example, by quantifying the amount of intact DNA present following restriction digestion with a methylation dependent restriction enzyme or by comparing amplification profiles after bisulfite reaction or by comparing sequences of bisulfite-treated and untreated DNA. Accordingly, a value, e.g., a methylation value, represents the methylation status and can thus be used as a quantitative indicator of methylation status across multiple copies of a locus. This is of particular use when it is desirable to compare the methylation status of a sequence in a sample to a threshold or reference value.

As used herein, the "sensitivity" of a given marker refers to the percentage of samples that report a DNA methylation value above a threshold value that distinguishes between neoplastic and non-neoplastic samples. In some embodiments, a positive is defined as a histology-confirmed neoplasia that reports a DNA methylation value above a threshold value (e.g., the range associated with disease), and a false negative is defined as a histology-confirmed neoplasia that reports a DNA methylation value below the threshold value (e.g., the range associated with no disease). The value of sensitivity, therefore, reflects the probability that a DNA methylation measurement for a given marker obtained from a known diseased sample will be in the range of disease-associated measurements. As defined here, the clinical relevance of the calculated sensitivity value represents an estimation of the probability that a given marker would detect the presence of a clinical condition when applied to a subject with that condition.

As used herein, the "specificity" of a given marker refers to the percentage of non-neoplastic samples that report a DNA methylation value below a threshold value that distinguishes between neoplastic and non-neoplastic samples. In some embodiments, a negative is defined as a histology-confirmed non-neoplastic sample that reports a DNA methylation value below the threshold value (e.g., the range associated with no disease) and a false positive is defined as a histology-confirmed non-neoplastic sample that reports a DNA methylation value above the threshold value (e.g., the range associated with disease). The value of specificity, therefore, reflects the probability that a DNA methylation measurement for a given marker obtained from a known non-neoplastic sample will be in the range of non-disease associated measurements. As defined here, the clinical relevance of the calculated specificity value represents an estimation of the probability that a given marker would detect the absence of a clinical condition when applied to a patient without that condition.

The term "gene" refers to a nucleic acid (e.g., DNA or RNA) sequence that comprises coding sequences necessary for the production of an RNA, or of a polypeptide or its precursor. A functional polypeptide can be encoded by a full length coding sequence or by any portion of the coding sequence as long as the desired activity or functional properties (e.g., enzymatic activity, ligand binding, signal transduction, etc.) of the polypeptide are retained. The term "portion" when used in reference to a gene refers to fragments of that gene. The fragments may range in size from a few nucleotides to the entire gene sequence minus one nucleotide. Thus, "a nucleotide comprising at least a portion of a gene" may comprise fragments of the gene or the entire gene.

The term "gene" also encompasses the coding regions of a structural gene and includes sequences located adjacent to the coding region on both the 5' and 3' ends for a distance of about 1 kb on either end such that the gene corresponds to the length of the full-length mRNA. The sequences which are located 5' of the coding region and which are present on the mRNA are referred to as 5' non-translated or untranslated sequences. The sequences which are located 3' or downstream of the coding region and which are present on the mRNA are referred to as 3' non-translated or 3' untranslated sequences. The term "gene" encompasses both cDNA and genomic forms of a gene. A genomic form or clone of a gene contains the coding region interrupted with non-coding sequences termed "introns" or "intervening regions" or "intervening sequences." Introns are segments of a gene which are transcribed into nuclear RNA (hnRNA); introns may contain regulatory elements such as enhancers. Introns are removed or "spliced out" from the nuclear or primary transcript; introns therefore are absent in the messenger RNA (mRNA) transcript. The mRNA functions during translation to specify the sequence or order of amino acids in a nascent polypeptide.

In addition to containing introns, genomic forms of a gene may also include sequences located on both the 5' and 3' end of the sequences which are present on the RNA transcript. These sequences are referred to as "flanking" sequences or regions (these flanking sequences are located 5' or 3' to the non-translated sequences present on the mRNA transcript). The 5' flanking region may contain regulatory sequences such as promoters and enhancers which control or influence the transcription of the gene. The 3' flanking region may contain sequences which direct the termination of transcription, posttranscriptional cleavage and polyadenylation.

As used herein, the term "bisulfite reagent" refers to a reagent comprising in some embodiments bisulfite, disulfite, hydrogen sulfite, or combinations thereof to distinguish between methylated and unmethylated cytidines, e.g., in CpG dinucleotide sequences.

The term "methylation assay" refers to any assay for determining the methylation state of one or more CpG dinucleotide sequences within a sequence of DNA.

As used herein, a "diagnostic" test application includes the detection or identification of a disease state or condition of a subject, determining the likelihood that a subject will contract a given disease or condition, determining the likelihood that a subject with a disease or condition will respond to therapy, determining the prognosis of a subject with a disease or condition (or its likely progression or regression), and determining the effect of a treatment on a subject with a disease or condition. For example, a diagnostic can be used for detecting the presence or likelihood of a subject contracting a colorectal neoplasm (e.g., CRC) or the likelihood that such a subject will respond favorably to a compound (e.g., a pharmaceutical, e.g., a drug) or other treatment.

The terms "oligonucleotide" or "polynucleotide" or "nucleotide" or "nucleic acid" refer to a molecule having two or more deoxyribonucleotides or ribonucleotides, preferably more than three, and usually more than ten. The exact size will depend on many factors, which in turn depends on the ultimate function or use of the oligonucleotide. The oligonucleotide may be generated in any manner, including chemical synthesis, DNA replication, reverse transcription, or a combination thereof.

The terms "an oligonucleotide having a nucleotide sequence encoding a gene" or "a nucleic acid sequence encoding" a specified polypeptide refer to a nucleic acid sequence comprising the coding region of a gene or, in other words, the nucleic acid sequence which encodes a gene product. The coding region may be present in a cDNA, a genomic DNA, or in an RNA. When present in a DNA form, the oligonucleotide may be single-stranded (i.e., the sense strand) or double-stranded. Suitable control elements such as enhancers/promoters, splice junctions, polyadenylation signals, etc. may be placed in close proximity to the coding region of the gene if needed to permit proper initiation of transcription and/or correct processing of the primary RNA transcript. Alternatively, the coding region utilized in the expression vectors of the present invention may contain endogenous enhancers/promoters, splice junctions, intervening sequences, polyadenylation signals, etc. or a combination of both endogenous and exogenous control elements.

The terms "complementary" and "complementarity" refer to polynucleotides (e.g., a sequence of nucleotides) related by the base-pairing rules. For example, the sequence 5'-A-G-T-3' is complementary to the sequence 3'-T-C-A-5'. Complementarity may be "partial," in which only some of the nucleic acids' bases are matched according to the base pairing rules. Or, there may be "complete" or "total" complementarity between the nucleic acids. The degree of complementarity between nucleic acid strands has significant effects on the efficiency and strength of hybridization between nucleic acid strands. This is of particular importance in amplification reactions, as well as detection methods that depend upon binding between nucleic acids.

The term "wild-type" when made in reference to a gene refers to a gene that has the characteristics of a gene isolated from a naturally occurring source. The term "wild-type" when made in reference to a gene product refers to a gene product that has the characteristics of a gene product isolated from a naturally occurring source. The term "naturally-occurring" as applied to an object refers to the fact that an object can be found in nature. For example, a polypeptide or polynucleotide sequence that is present in an organism (including viruses) that can be isolated from a source in nature and which has not been intentionally modified by the hand of a person in the laboratory is naturally-occurring. A wild-type gene is often that gene or allele which is most frequently observed in a population and is thus arbitrarily designated the "normal" or "wild-type" form of the gene. In contrast, the term "modified" or "mutant" when made in reference to a gene or to a gene product refers, respectively, to a gene or to a gene product that displays modifications in sequence and/or functional properties (i.e., altered characteristics) when compared to the wild-type gene or gene product. It is noted that naturally-occurring mutants can be isolated; these are identified by the fact that they have altered characteristics when compared to the wild-type gene or gene product.

The term "allele" refers to a variation of a gene; the variations include but are not limited to variants and mutants, polymorphic loci and single nucleotide polymorphic loci, frameshift and splice mutations. An allele may occur naturally in a population or it might arise during the lifetime of any particular individual of the population.

Thus, the terms "variant" and "mutant" when used in reference to a nucleotide sequence refer to a nucleic acid sequence that differs by one or more nucleotides from another, usually related, nucleotide acid sequence. A "variation" is a difference between two different nucleotide sequences; typically, one sequence is a reference sequence.

As used herein, the term "adenoma" refers to a benign tumor of glandular origin. Although these growths are benign, over time they may progress to become malignant. As used herein the term "colorectal adenoma" refers to a benign colorectal tumor in which the cells form recognizable glandular structures or in which the cells are clearly derived from glandular epithelium.

"Amplification" is a special case of nucleic acid replication involving template specificity. It is to be contrasted with non-specific template replication (i.e., replication that is template-dependent but not dependent on a specific template). Template specificity is here distinguished from fidelity of replication (i.e., synthesis of the proper polynucleotide sequence) and nucleotide (ribo- or deoxyribo-) specificity. Template specificity is frequently described in terms of "target" specificity. Target sequences are "targets" in the sense that they are sought to be sorted out from other nucleic acid. Amplification techniques have been designed primarily for this sorting out.

Amplification of nucleic acids generally refers to the production of multiple copies of a polynucleotide, or a portion of the polynucleotide, typically starting from a small amount of the polynucleotide (*e.g.,* a single polynucleotide molecule), where the amplification products or amplicons are generally detectable. Amplification of polynucleotides encompasses a variety of chemical and enzymatic processes. The generation of multiple DNA copies from one or a few copies of a target or template DNA molecule during a polymerase chain reaction (PCR) or a ligase chain reaction (LCR; see, *e.g.,* U.S. Patent No. 5,494,810) are forms of amplification. Additional types of amplification include, but are not limited to, allele-specific PCR (see, *e.g.,* U.S. Patent No. 5,639,611), assembly PCR (see, *e.g.,* U.S. Patent No. 5,965, 408), helicase-dependent amplification (see, *e.g.,* U.S. Patent No. 7,662,594), Hot-start PCR (see, *e.g.,* U.S. Patent Nos. 5,773,258 and 5,338,671), intersequence-specfic PCR, inverse PCR (see, *e.g.,* Triglia, et alet al. (1988) Nucleic Acids Res., 16:8186), ligation-mediated PCR (see, *e.g.,* Guilfoyle, R. et alet al., Nucleic Acids Research, 25:1854-1858 (1997); U.S. Patent No. 5,508,169), methylation-specific PCR (see, *e.g.,* Herman, et al., (1996) PNAS 93(13) 9821-9 826), miniprimer PCR, multiplex ligation-dependent probe amplification (see, *e.g.,* Schouten, et al., (2002) Nucleic Acids Research 30(12): e57), multiplex PCR (see, *e.g.,* Chamberlain, et al., (1988) Nucleic Acids Research 16(23) 11141-11156; Ballabio, et al., (1990) Human Genetics 84(6) 571-573; Hayden, et al., (2008) BMC Genetics 9:80), nested PCR, overlap-extension PCR (see, *e.g.,* Higuchi, et al., (1988) Nucleic Acids Research 16(15) 7351-7367), real time PCR (see, *e.g.,* Higuchi, et alet al., (1992) Biotechnology 10:413-417; Higuchi, et al., (1993) Biotechnology 11:1026-1030), reverse transcription PCR (see, *e.g.,* Bustin, S.A. (2000) J. Molecular Endocrinology 25:169-1 93), solid phase PCR, thermal asymmetric interlaced PCR, and Touchdown PCR (see, *e.g.,* Don, et al., Nucleic Acids Research (1991) 19(14) 4008; Roux, K. (1994) Biotechniques 16(5) 812-814; Hecker, et al., (1996) Biotechniques 20(3) 478-485). Polynucleotide amplification also can be accomplished using digital PCR (see, *e.g.,* Kalinina, et al., Nucleic Acids Research. 25; 1999-2004, (1997); Vogelstein and Kinzler, Proc Natl Acad Sci USA. 96; 9236-41, (1999); International Patent Publication No. WO05023091A2; US Patent Application Publication No. 20070202525). The term "polymerase chain reaction" ("PCR") refers to the method of K.B. Mullis U.S. Patent Nos. 4,683,195, 4,683,202, and 4,965,188, that describe a method for increasing the concentration of a segment of a target sequence in a mixture of genomic DNA without cloning or purification. This process for amplifying the target sequence consists of introducing a large excess of two oligonucleotide primers to the DNA mixture containing the desired target sequence, followed by a precise sequence of thermal cycling in the presence of a DNA polymerase. The two primers are complementary to their respective strands of the double stranded target sequence. To effect amplification, the mixture is denatured and the primers then annealed to their complementary sequences within the target molecule. Following annealing, the primers are extended with a polymerase so as to form a new pair of complementary strands. The steps of denaturation, primer annealing, and polymerase extension can be repeated many times (*i.e.,* denaturation, annealing and extension constitute one "cycle"; there can be numerous "cycles") to obtain a high concentration of an amplified segment of the desired target sequence. The length of the amplified segment of the desired target sequence is determined by the relative positions of the primers with respect to each other, and therefore, this length is a controllable parameter. By virtue of the repeating aspect of the process, the method is referred to as the "polymerase chain reaction" ("PCR"). Because the desired amplified segments of the target sequence become the predominant sequences (in terms of concentration) in the mixture, they are said to be "PCR amplified" and are "PCR products" or "amplicons."

Template specificity is achieved in most amplification techniques by the choice of enzyme. Amplification enzymes are enzymes that, under conditions they are used, will process only specific sequences of nucleic acid in a heterogeneous mixture of nucleic acid. For example, in the case of Q-beta replicase, MDV-1 RNA is the specific template for the replicase (Kacian et al., Proc. Natl. Acad. Sci. USA, 69:3038 [1972]). Other nucleic acid will not be replicated by this amplification enzyme. Similarly, in the case of T7 RNA polymerase, this amplification enzyme has a stringent specificity for its own promoters (Chamberlin et al, Nature, 228:227 [1970]). In the case of T4 DNA ligase, the enzyme will not ligate the two oligonucleotides or polynucleotides, where there is a mismatch between the oligonucleotide or polynucleotide substrate and the template at the ligation junction (Wu and Wallace, Genomics, 4:560 [1989]). Finally, thermostable template-dependant DNA polymerases (e.g., Taq and Pfu DNA polymerases), by virtue of their ability to function at high temperature, are found to display high specificity for the sequences bounded and thus defined by the primers; the high temperature results in thermodynamic conditions that favor primer hybridization with the target sequences and not hybridization with non-target sequences (H. A. Erlich (ed.), PCR Technology, Stockton Press [1989]).

As used herein, the term "nucleic acid detection assay" refers to any method of determining the nucleotide composition of a nucleic acid of interest. Nucleic acid detection assay include but are not limited to, DNA sequencing methods, probe hybridization methods, structure specific cleavage assays (*e.g.,* the INVADER assay, (Hologic, Inc.) and are described, *e.g.,* in U.S. Patent Nos. 5,846,717, 5,985,557, 5,994,069, 6,001,567, 6,090,543, and 6,872,816; Lyamichev et al., Nat. Biotech., 17:292 (1999), Hall et al., PNAS, USA, 97:8272 (2000), and US 2009/0253142); enzyme mismatch cleavage methods (e.g., Variagenics, U.S. Pat. Nos. 6,110,684, 5,958,692, 5,851,770); polymerase chain reaction; branched hybridization methods (e.g., Chiron, U.S. Pat. Nos. 5,849,481, 5,710,264, 5,124,246, and 5,624,802); rolling circle replication (e.g., U.S. Pat. Nos. 6,210,884, 6,183,960 and 6,235,502); NASBA (e.g., U.S. Pat. No. 5,409,81 8); molecular beacon technology (e.g., U.S. Pat. No. 6,150,097); E-sensor technology (Motorola, U.S. Pat. Nos. 6,248,229, 6,221,583, 6,013,170, and 6,063, 573); cycling probe technology (e.g., U.S. Pat. Nos. 5,403,711, 5,011,769, and 5,660,988);

Dade Behring signal amplification methods (e.g., U.S. Pat. Nos. 6,121,001, 6,110,677, 5,914,230, 5,882,867, and 5,792,614); ligase chain reaction (e.g., Barnay Proc. Natl. Acad. Sci USA 88, 189-93 (1991)); and sandwich hybridization methods (e.g., U.S. Pat. No. 5,28 8,609).

The term "amplifiable nucleic acid" refers to a nucleic acid that may be amplified by any amplification method. It is contemplated that "amplifiable nucleic acid" will usually comprise "sample template."

The term "sample template" refers to nucleic acid originating from a sample that is analyzed for the presence of "target" (defined below). In contrast, "background template" is used in reference to nucleic acid other than sample template that may or may not be present in a sample. Background template is most often inadvertent. It may be the result of carryover or it may be due to the presence of nucleic acid contaminants sought to be purified away from the sample. For example, nucleic acids from organisms other than those to be detected may be present as background in a test sample.

The term "primer" refers to an oligonucleotide, whether occurring naturally as in a purified restriction digest or produced synthetically, that is capable of acting as a point of initiation of synthesis when placed under conditions in which synthesis of a primer extension product that is complementary to a nucleic acid strand is induced, (e.g., in the presence of nucleotides and an inducing agent such as a DNA polymerase and at a suitable temperature and pH). The primer is preferably single stranded for maximum efficiency in amplification, but may alternatively be double stranded. If double stranded, the primer is first treated to separate its strands before being used to prepare extension products. Preferably, the primer is an oligodeoxyribonucleotide. The primer must be sufficiently long to prime the synthesis of extension products in the presence of the inducing agent. The exact lengths of the primers will depend on many factors, including temperature, source of primer, and the use of the method.

The term "probe" refers to an oligonucleotide (e.g., a sequence of nucleotides), whether occurring naturally as in a purified restriction digest or produced synthetically, recombinantly, or by PCR amplification, that is capable of hybridizing to another oligonucleotide of interest. A probe may be single-stranded or double-stranded. Probes are useful in the detection, identification, and isolation of particular gene sequences (e.g., a "capture probe"). It is contemplated that any probe used in the present invention may, in some embodiments, be labeled with any "reporter molecule," so that is detectable in any detection system, including, but not limited to enzyme (e.g., ELISA, as well as enzyme-based histochemical assays), fluorescent, radioactive, and luminescent systems. It is not intended that the present invention be limited to any particular detection system or label.

The term "target," as used herein refers to a nucleic acid sought to be sorted out from other nucleic acids, *e.g.,* by probe binding, amplification, isolation, capture, ***etc.*** For example, when used in reference to the polymerase chain reaction, "target" refers to the region of nucleic acid bounded by the primers used for polymerase chain reaction, while in an invasive cleavage assay, a target comprises the site at which a probe and invasive oligonucleotides (e.g., INVADER oligonucleotide) bind to form an invasive cleavage structure, such that the presence of the target nucleic acid can be detected. A "segment" is defined as a region of nucleic acid within the target sequence.

The term "isolated" when used in relation to a nucleic acid, as in "an isolated oligonucleotide" refers to a nucleic acid sequence that is identified and separated from at least one contaminant nucleic acid with which it is ordinarily associated in its natural source. Isolated nucleic acid is present in a form or setting that is different from that in which it is found in nature. In contrast, non-isolated nucleic acids, such as DNA and RNA, are found in the state they exist in nature. Examples of non-isolated nucleic acids include: a given DNA sequence (e.g., a gene) found on the host cell chromosome in proximity to neighboring genes; RNA sequences, such as a specific mRNA sequence encoding a specific protein, found in the cell as a mixture with numerous other mRNAs which encode a multitude of proteins. However, isolated nucleic acid encoding a particular protein includes, by way of example, such nucleic acid in cells ordinarily expressing the protein, where the nucleic acid is in a chromosomal location different from that of natural cells, or is otherwise flanked by a different nucleic acid sequence than that found in nature. The isolated nucleic acid or oligonucleotide may be present in single-stranded or double-stranded form. When an isolated nucleic acid or oligonucleotide is to be utilized to express a protein, the oligonucleotide will contain at a minimum the sense or coding strand (i.e., the oligonucleotide may be single-stranded), but may contain both the sense and anti-sense strands (i.e., the oligonucleotide may be double-stranded). An isolated nucleic acid may, after isolation from its natural or typical environment, by be combined with other nucleic acids or molecules. For example, an isolated nucleic acid may be present in a host cell in which into which it has been placed, e.g., for heterologous expression.

The term "purified" refers to molecules, either nucleic acid or amino acid sequences, that are removed from their natural environment, isolated, or separated. An "isolated nucleic acid sequence" may therefore be a purified nucleic acid sequence. "Substantially purified" molecules are at least 60% free, preferably at least 75% free, and more preferably at least 90% free from other components with which they are naturally associated. As used herein, the terms "purified" or "to purify" also refer to the removal of contaminants from a sample. The removal of contaminating proteins results in an increase in the percent of polypeptide or nucleic acid of interest in the sample. In another example, recombinant polypeptides are expressed in plant, bacterial, yeast, or mammalian host cells and the polypeptides are purified by the removal of host cell proteins; the percent of recombinant polypeptides is thereby increased in the sample.

The term "composition comprising" a given polynucleotide sequence or polypeptide refers broadly to any composition containing the given polynucleotide sequence or polypeptide. The composition may comprise an aqueous solution containing salts (e.g., NaCl), detergents (e.g., SDS), and other components (e.g., Denhardt's solution, dry milk, salmon sperm DNA, etc.).

The term "sample" is used in its broadest sense. In one sense it can refer to an animal cell or tissue. In another sense, it is meant to include a specimen or culture obtained from any source, as well as biological and environmental samples. Biological samples may be obtained from plants or animals (including humans) and encompass fluids, solids, tissues, and gases. Environmental samples include environmental material such as surface matter, soil, water, and industrial samples. These examples are not to be construed as limiting the sample types applicable to the present invention.

As used herein, the terms "patient" or "subject" refer to organisms to be subject to various tests provided by the technology. The term "subject" includes animals, preferably mammals, including humans. In a preferred embodiment, the subject is a primate. In an even more preferred embodiment, the subject is a human.

As used herein, the term "kit" refers to any delivery system for delivering materials. In the context of reaction assays, such delivery systems include systems that allow for the storage, transport, or delivery of reaction reagents (*e.g.,* oligonucleotides, enzymes, *etc.* in the appropriate containers) and/or supporting materials (*e.g*., buffers, written instructions for performing the assay *etc*.) from one location to another. For example, kits include one or more enclosures (e.g., boxes) containing the relevant reaction reagents and/or supporting materials. As used herein, the term "fragmented kit" refers to a delivery systems comprising two or more separate containers that each contain a subportion of the total kit components. The containers may be delivered to the intended recipient together or separately. For example, a first container may contain an enzyme for use in an assay, while a second container contains oligonucleotides. The term "fragmented kit" is intended to encompass kits containing Analyte specific reagents (ASR's) regulated under section 520(e) of the Federal Food, Drug, and Cosmetic Act, but are not limited thereto. Indeed, any delivery system comprising two or more separate containers that each contains a subportion of the total kit components are included in the term "fragmented kit." In contrast, a "combined kit" refers to a delivery system containing all of the components of a reaction assay in a single container (*e.g.,* in a single box housing each of the desired components). The term "kit" includes both fragmented and combined kits.

### Embodiments of the technology

### Genes of the marker panel

*NDRG4, BMP3, vimentin,* and *TFPI2* were selected from a larger set of nine candidate genes that individually or in combination yielded nearly complete separation of colorectal neoplasia from normal mucosa. *NDRG4, BMP3, vimentin,* and *TFPI2* were the most discriminant of these nine candidates. Aberrant methylation of the SEPT9 gene highly discriminates colorectal neoplasia from normal mucosa at the tissue level, and thus the level of methylated SEPT9 is also included in the gene panel. In addition, both mutant *KRAS* and hemoglobin complement these markers and, accordingly, were also evaluated in some embodiments of the methods provided.

In some embodiments, beta-actin (*ACTB*) was assayed as a reference gene to determine total human genome equivalents in stool. Using beta-actin as a reference allowed the assessment of sample adequacy (e.g., samples with less than 75 copies per reaction volume were rejected to prevent stochastic error), normalization of marker levels (the beta-actin level served as denominator for marker ratios), and evaluation of human DNA level as a candidate marker itself, as total human DNA increases with colorectal neoplasia.

*Vimentin* (VIM) encodes a member of the intermediate filament family. Intermediate filamentents, along with microtubules and actin microfilaments, make up the cytoskeleton. The protein encoded by the *vimentin* gene is responsible for maintaining cell shape, integrity of the cytoplasm, and stabilizing cytoskeletal interactions. It is also involved in the immune response, and controls the transport of low-density lipoprotein-derived cholesterol from a lysosome to the site of esterification. It functions as an organizer of a number of critical proteins involved in attachment, migration, and cell signaling. *Vimentin* is located on human chromosome 10 at 10p13, has the locus tag RP11-124N14.1, and the human gene sequence is provided by NCBI accession number NG_012413.1.

*NDRG4* (NDRG family member 4, also known as *BDM1* and *SMAP-8*) is a member of the N-myc downregulated gene family, which belongs to the alpha/beta hydrolase superfamily. The protein encoded by the *NDRG4* gene is a cytoplasmic protein that is required for cell cycle progression and survival in primary astrocytes and may be involved in the regulation of mitogenic signalling in vascular smooth muscle cells. Accordingly, the protein is known variously as smooth muscle-associated protein 8, brain development-related molecule 1, and vascular smooth muscle cell-associated protein 8. Alternative splicing results in multiple transcripts encoding different isoforms. *NDRG4* is located on human chromosome 16 at 16q21-q22.1 and the human gene sequence is provided by NCBI accession number NC_000016.9 (at bases 58,497,549 to 58,547,523).

*BMP3* (bone morphogenetic protein 3, also known as BMP-3A) belongs to the transforming growth factor-beta (TGFB) superfamily. Bone morphogenic protein, also known as osteogenin, induces bone formation. *BMP3* is located on human chromosome 4 at 4q21 and the human gene sequence is provided by NCBI accession number NC_000004.11 (at bases 81,952,119 to 81,978,685).

*TFPI2* (tissue factor pathway inhibitor 2, also known as PP5, REF1, and TFPI-2) is on human chromosome 7 at 7q22 and the human gene sequence is provided by NCBI accession number NC_000007.13 (at bases 93,515,745 to 93,520,065, complement).

SEPT9 (septin 9, also known as MSF, MSF1, NAPB, SINT1, PNUTL4, SeptD1, and AF 17q25) is a member of the septin family involved in cytokinesis and cell cycle control. SEPT9 is a candidate for the ovarian tumor suppressor gene. Mutations in this gene cause hereditary neuralgic amyotrophy, also known as neuritis with brachial predilection. A chromosomal translocation involving this gene on chromosome 17 and the MLL gene on chromosome 11 results in acute myelomonocytic leukemia. Multiple alternatively spliced transcript variants encoding different isoforms have been described. It is present at 17q25 on human chromosome 17 and the human gene sequence is provided by NCBI accession number NG_011683.

*KRAS* (v-Ki-ras2 Kirsten rat sarcoma viral oncogene homolog, also known as NS, NS3, KRAS1, KRAS2, RASK2, KI-RAS, C-K-RAS, K-RAS2A, K-RAS2B, K-RAS4A, K-RAS4B) is a Kirsten ras oncogene homolog from the mammalian ras gene family. *KRAS* encodes a protein that is a member of the small GTPase superfamily. In some mutant forms of *KRAS,* a single amino acid substitution is responsible for an activating mutation. The transforming protein that results is implicated in various malignancies, including lung adenocarcinoma, mucinous adenoma, ductal carcinoma of the pancreas, and colorectal carcinoma. Alternative splicing leads to variants encoding two isoforms that differ in the C-terminal region. It is present on human chromosome 12 at 12p12.1 and has the human gene sequence is provided by NCBI accession number NG_007524.

### DNA methylation

The methylation states of the *vimentin, NDRG4, BMP3, TFPI3,* and SEPT9 genes have been associated with colorectal cancer. DNA methylation is an epigenetic modification that regulates gene expression and marks imprinted genes. Consequently, aberrant DNA methylation is known to disrupt embryonic development and cell cycle regulation, and it can promote oncogenesis that produces cancers.

Specifically, the aberrant methylation of the *Septin 9* gene highly discriminates colorectal neoplasia from normal mucosa at the tissue level (Lofton-Day C, et al. "DNA methylation biomarkers for blood-based colorectal cancer screening", Clin Chem 2008; 54: 41 4-23). Assay of naked methylated *Septin 9* in plasma has been shown to detect CRC. In particular, SEPT9 yielded sensitivities for CRC ranging from 52-73% at specificities ranging from 84-91%; CRC detection rates were higher with late than with early stages (see, e.g., Lofton-Day C, et al. "DNA methylation biomarkers for blood-based colorectal cancer screening", Clin Chem 2008; 54: 414-23; Grutzmann R, et al. "Sensitive detection of colorectal cancer in peripheral blood by septin 9 DNA methylation assay", PLoS One 2008; 3: e3759; deVos T, et al. "Circulating methylated SEPT9 DNA in plasma is a biomarker for colorectal cancer", Clin Chem 2009; 55: 1337-46; Tanzer M, et al. "Performance of epigenetic markers SEPT9 and ALX4 in plasma for detection of colorectal precancerous lesions", PLoS One 2010; 5: e9061).

Using an analytically modified assay, SEPT9 detected 90% of referred CRC at 89% specificity in a preliminary report (Heichman KA, et al. "Use of Septin 9 methylated DNA biomarker to detect cancer in the blood of colorectal cancer patients", Presented at ASCO-NCI-EORTC meeting in Philadelphia. October, 2010). And, preliminary data from a large study on average-risk persons suggest a CRC detection rate of 50% by SEPT9; post-hoc analyses using a more sensitive assay algorithm suggest a CRC sensitivity of 67% and specificity of 88% (Church TR, et al. "Prospective Clinical Validation of an Assay for Methylated SEPT9 DNA in Human Plasma as a Colorectal Cancer Screening Tool in Average Risk Men and Women ≥50 Years", Gastroenterology 2010; 130: e18). Detection rates of advanced adenomas by SEPT9 have been reported in two studies (Grutzmann R, et al. "Sensitive detection of colorectal cancer in peripheral blood by septin 9 DNA methylation assay", PLoS One 2008; 3: e3759; Tanzer M, et al. "Performance of epigenetic markers SEPT9 and ALX4 in plasma for detection of colorectal precancerous lesions", PLoS One 2010; 5: e906 1), and sensitivities were 17% (3 positive of 18) and 18% (3 positive of 17). The SEPT9 assay is now commercially available as a laboratory developed test.

Luminal exfoliation logically occurs earlier than vascular invasion in the course of colorectal tumorigenesis. To complement the assay of methylated *Septin 9* in plasma, assessing additional markers in stool DNA detects precancerous lesions in the digestive tract that have not invaded the basement membrane and/or released DNA markers into the blood. Next-generation stool DNA (sDNA) testing provides high detection rates of both CRC and precancers buoyed by key technical advances including use of a preservative buffer with stool collection (Olson J, et al. "DNA stabilization is critical for maximizing performance of fecal DNA-based colorectal cancer tests", Diagn Mol Pathol 2005; 14: 183-91; Zou H, et al. "A sensitive method to quantify human long DNA in stool: relevance to colorectal cancer screening", Cancer Epidemiol Biomarkers Prev 2006; 15: 1115-9), highly discriminant marker panels (Zou H, et al. "Sensitive quantification of methylated markers with a novel methylation specific technology", Clin Chem 2010; 56: A199), and assays with analytical sensitivity one hundred to a thousand times greater than conventional approaches (Zou H AH, et al. "Sensitive quantification of methylated markers with a novel methylation specific technology", Clin Chem 2010; 56: A199; Zou H, et al. "High detection rates of colorectal neoplasia by stool DNA testing with a novel digital melt curve assay", Gastroenterology 2009; 136: 459-70; Diehl F, et al. "Analysis of mutations in DNA isolated from plasma and stool of colorectal cancer patients", Gastroenterology 2008; 135: 489-98. This approach is biologically based on exfoliation, which occurs continuously from the luminal surface of precancers and all stages of cancer (Ahlquist DA. "Molecular detection of colorectal neoplasia", Gastroenterology 2010; 138: 2127-39; Loktionov A, et al. "Quantitation of DNA from exfoliated colonocytes isolated from human stool surface as a novel noninvasive screening test for colorectal cancer", Clin Cancer Res 1998; 4: 337-42; Ahlquist DA, et al. "Morphometric analysis of the 'mucocellular layer' overlying colorectal cancer and normal mucosa: relevance to exfoliation and stool screening", Hum Pathol 2000; 31: 51 -7).

A number of small studies using next-generation techniques have demonstrated that assay of methylated and mutated gene markers in stool can achieve high clinical sensitivity for both CRC and advanced adenomas (Zou H, et al. "High detection rates of colorectal neoplasia by stool DNA testing with a novel digital melt curve assay", Gastroenterology 2009; 136: 459-70; Itzkowitz SH, et al. "Improved fecal DNA test for colorectal cancer screening", Clin Gastroenterol Hepatol 2007; 5: 111-7; Zou H, et al. "Quantitative stool DNA testing for detection of both colorectal cancer and advanced adenoma", Gastroenterology (on-line) 2009; 136 (5 Suppl 1): A 625). In a large case-control study, a prototype multi-marker sDNA test detected 85% of CRCs and 63% of adenomas greater than 1 cm at a specificity cutoff of 90%; detection rates rose with increasing neoplasm size but were unaffected by neoplasm site or stage (Ahlquist DA, et al. "Next Generation Stool DNA Test Accurately Detects Colorectal Cancer and Large Adenomas", Gastroenterology 2012).

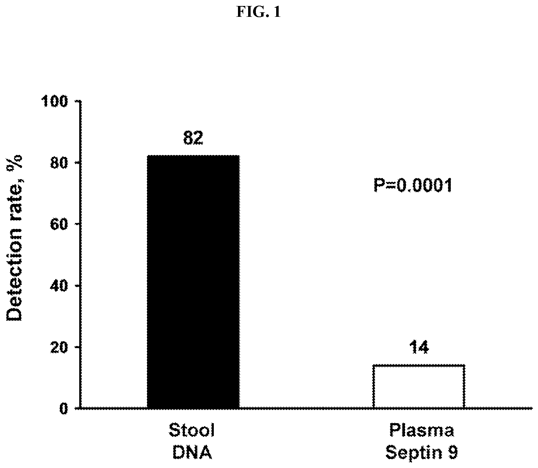

In some instances the examination of a panel of DNA markers in stool samples fails to detect colorectal adenomas or CRC with 100% sensitivity, even when additional markers are included with a panel. For example, the sensitivity of analysis of stool DNA for methylation status of a panel of markers (*BMP3, NDRG4, vimentin,* and *TFPI2*) has been compared to the sensitivity of the SEPT9 assay of the Septin 9 marker in plasma (Ahlquist DA, et al. Clinical Gastroenterology and Hepatology, vol. 10, Pages 272-277.el, March 2012). In this study, the sDNA test had a detection rate of 82%, compared to a 14% detection rate for the plasma Septin 9 test (Ahlquist, et al., *supra*). As shown in Figure 1, the sDNA test showed a higher detection rate over all and for colorectal cancers in stages I-III. Only in advanced stage IV did the plasma SEPT9 assay show a higher detection rate.

The present invention provides an improved test comprising combining the data from paired samples prior to analysis. As discussed below, when Septin 9 data and sDNA marker panel data from paired cancer samples are analyzed together, the overall sensitivity of detection is substantially improved. Shown below in Tables 1-3 are the results of analyzing paired cancer and adenoma samples using the sDNA panel and the plasma Septin 9 assay (see the Example, below).

**TABLE 1 - sDNA and SEPT9 combined panel for cancer**

| Patient No | **sDNAassay** | **Plasma assay** | **Combined result** |
|---|---|---|---|
| 1 | Positive | Positive | Positive |
| 2 | Positive | Positive | Positive |
| 3 | Positive | Positive | Positive |
| 4 | Positive | Positive | Positive |
| 5 | Positive | Positive | Positive |
| 6 | Positive | Positive | Positive |
| 7 | Positive | Positive | Positive |
| 8 | Positive | Positive | Positive |
| 9 | Positive | Positive | Positive |
| 10 | Positive | Positive | Positive |
| 11 | Positive | Positive | Positive |
| 12 | Positive | Positive | Positive |
| 13 | Positive | Positive | Positive |
| 14 | Positive | Positive | Positive |
| 15 | Positive | *Negative* | Positive |
| 16 | Positive | *Negative* | Positive |
| 17 | Positive | *Negative* | Positive |
| 18 | Positive | *Negative* | Positive |
| 19 | Positive | *Negative* | Positive |
| 20 | Positive | *Negative* | Positive |
| 21 | Positive | *Negative* | Positive |
| 22 | Positive | *Negative* | Positive |
| 23 | Positive | *Negative* | Positive |
| 24 | Positive | *Negative* | Positive |
| 25 | Positive | *Negative* | Positive |
| 26 | Positive | *Negative* | Positive |
| 27 | *Negative* | Positive | Positive |
| 28 | *Negative* | Positive | Positive |
| 29 | *Negative* | Positive | Positive |
| 30 | *Negative* | Positive | Positive |
| Sensitivity | 87% | 60% | 100% |

Table 1 demonstrates the improvement in sensitivity for cancer detection when paired stool and plasma samples are analyzed for each patient. These data are summarized in Table 2, below.

**TABLE 2 - Summary of sDNA and SEPT9 combined panel for cancer**

| **Cancer** Sensitivity All Matched Samples with | | SEPT9 test | | Total |
|---|---|---|---|---|
| | | Negative | Positive | |
| sDNA test | Negative | 0 | 4 | 4 |
| | Positive | 12 | 14 | 26 |
| | | | | Sensitivity: 87% |
| | | | | 95% CI: (69%, 96%) |
| Total | | 12 | 18 | 30 |
| | | | Sensitivity: 60% | |
| | | | 95% CI: (41%, 77%) | |

| | | | | |
|---|---|---|---|---|
| 68% Discordance; 100% of cases missed by Sept9 were called positive with sDNA test; 100% of cases missed by Prototype I were called positive with Sept9; The estimated sensitivity for the sDNA test alone was 87%, compared to 60% with plasma Sept9 alone (p=0.0455). | | | | |

**TABLE 3 - sDNA and SEPT9 combined panel for adenoma**

| **Adenoma** Sensitivity All Matched Samples with | | SEPT9 test | | Total |
|---|---|---|---|---|
| | | Negative | Positive | |
| sDNA test | Negative | 4 | 0 | 4 |
| | Positive | 15 | 3 | 18 |
| | | | | Sensitivity: 82% |
| | | | | 95% CI: (60%, 95%) |
| Total | | 19 | 3 | 22 |
| | | | Sensitivity: 14% | |
| | | | 95% CI: (3%, 35%) | |

| | | | | |
|---|---|---|---|---|
| 68% Discordance; 79% of cases missed by Sept9 were called positive with sDNA test; 0% of cases missed by sDNA test were called positive with Sept9; The estimated sensitivity for sDNA was 83% compared to 14% with Sept9 (p=0.0001) | | | | |

Table 3 demonstrates the improvement in sensitivity of adenoma detection when paired stool and plasma samples are analyzed for each patient.

### Measuring DNA methylation

In mammals, methylation occurs only at cytosine residues and more specifically only on a cytosine residue that is adjacent to a guanine residue (that is, at the sequence CG, often denoted "CpG"). Detecting and mapping sites of DNA methylation are essential steps for understanding epigenetic gene regulation and providing diagnostic tools for identifying cancers and other disease states associated with errors in gene regulation.

Mapping the state of DNA methylation at particular sites is currently accomplished by the bisulfite method described by Frommer, et al. for the detection of 5-methylcytosines in DNA (Proc. Natl. Acad. Sci. USA 89: 1827-31 (1992)) or variations thereof. The bisulfite method of mapping 5-methylcytosines is based on the observation that cytosine, but not 5-methylcytosine, reacts with hydrogen sulfite ion (also known as bisulfite). The reaction is usually performed according to the following steps: first, cytosine reacts with hydrogen sulfite to form a sulfonated cytosine. Next, spontaneous deamination of the sulfonated reaction intermediate results in a sulfonated uracil. Finally, the sulfonated uricil is desulfonated under alkaline conditions to form uracil. Detection is possible because uracil forms base pairs with adenine (thus behaving like thymine), whereas 5-methylcytosine base pairs with guanine (thus behaving like cytosine). This makes the discrimination of methylated cytosines from non-methylated cytosines possible by, e.g., bisulfite genomic sequencing (Grigg G, & Clark S, Bioessays (1994) 16: 431-36; Grigg G, DNA Seq. (1996) 6: 189-98) or methylation-specific PCR (MSP) as is disclosed, e.g., in U.S. Patent No. 5,786,146.

A gene's methylation state is often expressed as the fraction or percentage of individual strands of DNA that are methylated at a particular site (e.g., at a single nucleotide or at a longer sequence of interest, e.g., up to a ∼100-bp subsequence of a DNA) relative to the total population of DNA in the sample comprising that particular site. Traditionally, the amount of unmethylated (e.g., native) gene is determined by PCR using calibrators. Then, a known amount of DNA is bisulphite treated and the resulting methylation-specific sequence is determined using either a real-time PCR or an equivalent exponential amplification, e.g., a QuARTS assay.

For example, conventional methods generally comprise generating a standard curve for the unmethylated target by using external standards. The standard curve is constructed from at least two points and relates the real-time Cₜ value for unmethylated DNA to known quantitative standards. Then, a second standard curve for the methylated target is constructed from at least two points and external standards. This second standard curve relates the Cₜ for methylated DNA to known quantitative standards. Next, the test sample Cₜ values are determined for the methylated and unmethylated populations and the genomic equivalents of DNA are calculated from the standard curves produced by the first two steps. The percentage of methylation at the site of interest is calculated from the amount of methylated DNAs relative to the total amount of DNAs in the population, e.g., (number of methylated DNAs) / (the number of methylated DNAs + number of unmethylated DNAs) × 100.

The technology provided herein is not restricted in the method by which a gene's methylation state is measured. For example, in some embodiments the methylation state is measured by a genome scanning methods. For example, one method involves restriction landmark genomic scanning (Kawai et al., Mol. Cell. Biol. 14:7421-7427, 1994) and another example involves methylation-sensitive arbitrarily primed PCR (Gonzalgo et al., Cancer Res. 57:594-599, 1997). In some embodiments, changes in methylation patterns at specific CpG sites are monitored by digestion of genomic DNA with methylation-sensitive restriction enzymes followed by Southern analysis of the regions of interest (digestion-Southern method). In some embodiments, analyzing changes in methylation patterns involves a PCR-based process that involves digestion of genomic DNA with methylation-sensitive restriction enzymes prior to PCR amplification (Singer-Sam et al., Nucl. Acids Res. 18:687, 1990). In addition, other techniques have been reported that utilize bisulfite treatment of DNA as a starting point for methylation analysis. These include methylation-specific PCR (MSP) (Herman et al. Proc. Natl. Acad. Sci. USA 93:9821-9826, 1992) and restriction enzyme digestion of PCR products amplified from bisulfite-converted DNA (Sadri and Hornsby, Nucl. Acids Res. 24:5058-5059, 1996; and Xiong and Laird, Nucl. Acids Res. 25:2532-2534, 1997). PCR techniques have been developed for detection of gene mutations (Kuppuswamy et al., Proc. Natl. Acad. Sci. 55 USA 88:1143-1147, 1991) and quantification of allelic-specific expression (Szabo and Mann, Genes Dev. 9:3097-3108, 1995; and Singer-Sam et al., PCR Methods Appl. 1:160-163, 1992). Such techniques use internal primers, which anneal to a PCR-generated template and terminate immediately 5' of the single nucleotide to be assayed. Methods using a "quantitative Ms-SNuPE assay"as described in U.S. Pat. No. 7,037,650 are used in some embodiments.

Although the disclosure herein refers to certain illustrated embodiments, it is to be understood that these embodiments are presented by way of example and not by way of limitation.

### EXPERIMENTAL EXAMPLES

### Methods

### Stool Collection and Storage

Stools were collected prior to bowel purgation and colonoscopy or more than 1 week after colonoscopy (but before neoplasm resection). Whole stools (minimum 36 grams) were collected in buckets mounted to the toilet seat. A preservative buffer was added, most often immediately after defecation; and buffered stools were archived at -80°C. However, the timing of buffer addition and pre-storage homogenization varied across participating centers.

### Stool Processing and Target Gene Capture

Promptly after thawing, buffered stools were homogenized with a shaker device and centrifuged. A 12-ml aliquot of stool supernatant was then treated with polyvinylpolypyrrolidone (PVPP, Crosby & Baker, Westport MA) at a concentration of 50 mg/ml. Direct capture of target gene sequences by hybridization with oligonucleotide probes was performed on supernatants. Briefly, 10 ml of PVPP-treated supernatant was denatured in 2.4 M guanidine isothiocyanate (Sigma, St. Louis MO) at 90°C for 10 min; 500 µg Sera-Mag carboxylate modified beads (ThermoFisher Scientific, Waltham MA) functionalized with each oligonucleotide capture probe were subsequently added to denatured stool supernatant and incubated at room temperature for one hour. Sera-Mag beads were collected on a magnetic rack, and washed for three times using 1× MOPS washing buffer (10 mM MOPS, 150 mM NaCl, pH 7.5), and then eluted out in 50 µl nuclease free water with 20 ng/µl tRNA (Sigma). The four selected methylated markers, *vimentin, NDRG4, BMP3,* and *TFPI2,* and one reference gene *ACTB* were captured together in one hybridization reaction; the mutation marker *KRAS* was sequentially captured in another hybridization reaction. Capture probes (IDT, Coralville IA) used had the following sequences:
/5AmMC6/-CTG TAG GTG CGG GTG GAC GTA GTC ACG TAG CTC CGG CTG GA-3' (SEQ ID NO: 1) for *vimentin*
/5AmMC6/-TCC CTC GCG CGT GGC TTC CGC CTT CTG CGC GGC TGG GGT GCC CGG TGG-3' (SEQ ID NO: 2) for *NDRG4*
/5AmMC6/-GCG GGA CAC TCC GAA GGC GCA AGG AG-3' (SEQ ID NO: 3) for *BMP3*
/5AmMC6/-CGC CTG GAG CAG AAA GCC GCG CAC CT-3' (SEQ ID NO: 4) for *TFPI2*
/5AmMC6/-CCT TGT CAC ACG AGC CAG TGT TAG TAC CTA CAC C-3' (SEQ ID NO: 5) for *ACTB*
/5AmMC6/-GGC CTG CTG AAA ATG ACT GAA TAT AAA CTT GTG GTA GTT GGA GC-3' (SEQ ID NO: 6) for *KRAS;* and
/5AmMC6/-CTC TAT TGT TGG ATC ATA TTC GTC CAC AAA ATG ATT CTG AAT TAG C-3' (SEQ ID NO: 7) for *KRAS.*

### Marker Assays

Methylation markers, the reference gene beta-actin, and *KRAS* mutations were quantified by the QuARTS method (quantitative allele-specific real-time target and signal amplification) as described, e.g., in Zou H et al. "Sensitive quantification of methylated markers with a novel methylation specific technology", Clin Chem 2010; 56: A1 99). See also US Pat. Appl. Ser Nos. 12/946,737, 12/946,745, 12/946,752, and 61 /548,639). In sum, three reactions sequentially occur in each QuARTS assay, including DNA amplification (reaction 1) and target probe cleavage (reaction 2) in the primary reaction; and FRET cleavage and fluorescent signal generation (reaction 3) in the secondary reaction. When target DNA is amplified with specific primers, a specific detection probe with a flap sequence loosely binds to the amplicon. The presence of the specific invasive oligonucleotide at the target binding site causes cleavase to release the flap sequence by cutting between the detection probe and the flap sequence. The flap sequence is complementary to a nonhairpin portion of a corresponding FRET cassette. Accordingly, the flap sequence functions as an invasive oligonucleotide on the FRET cassette and effects a cleavage between the FRET cassette fluorophore and a quencher, which produces a fluorescent signal. The cleavage reaction can cut multiple probes per target and thus release multiple fluorophore per flap, providing exponential signal amplification. QuARTS can detect multiple targets in a single reaction well by using FRET cassettes with different dyes.

### Methylation Assays

First, 45 µl of captured DNA was treated with bisulfite using the EZ-96 DNA Methylation Kit according to manufacturer's instructions (Zymo Research, Irvine CA) and the sample was eluted in 50 µl of Zymo M-dilution buffer with 20 ng/µl tRNA (Sigma) on a 96-well PCR plate. Then, 10 µl of bisulfite-treated DNA was added to the QuARTS assays, which were set up in 30 µl total reaction volumes on a 96-well PCR plates. PCR plates were cycled in a LightCycler 480 (Roche). Two separate triplex QuARTS assays were designed to detect the methylated markers *vimentin, NDRG4, BMP3,* and *TFPI2* using *ACTB* as a reference gene for each assay. The first triplex assay contained *ACTB, vimentin,* and *NDRG4,* and the second contained *ACTB, BMP3,* and *TFPI2.* Each QuARTS reaction incorporated 400-600 nM primers and detection probes, 100 nM invasive oligo, 600-700 nM each of FAM (Hologic, Madison WI), Yellow (Hologic), and Quasar® 670 (BioSearch Technologies, Novato CA) fluorescence resonance energy transfer reporter cassettes (FRETs), 6.675 ng/µl Cleavase® 2.0 (Hologic), 1 unit hot-start GoTaq® DNA polymerase (Promega, Madison WI), 10 mM MOPS, 7.5 mM MgCl₂, and 250 µM of deoxyribonucleotide triphosphate (dNTP).

QuARTS cycling conditions consisted of 95 °C 3 min, 10 cycles at 95 °C 20 sec, 67 °C 30 sec, and 70 °C 30 sec, 45 cycles at 95 °C 20 sec, 53 °C 1 min, and 70 °C 30 sec, and 40 °C hold 30 sec. Methylation-specific primers (first two sequences in each set) and probes (third sequence in each set) were:
For *vimentin*
   5'-GGC GGT TCG GGT ATC G-3'(SEQ ID NO: 8)
   5'-CGT AAT CAC GTA ACT CCG AC T-3'(SEQ ID NO: 9)
   5'-GAC GCG GAG GCG AGT CGG TCG/3'C6/(SEQ ID NO: 10)
For *NDRG4*
   5'-CGG TTT TCG TTC GTT TTT TCG-3'(SEQ ID NO: 11)
   5'-GTA ACT TCC GCC TTC TAC GC-3'(SEQ ID NO: 12)
   5'-CGC CGA GGG TTC GTT TAT CG/3'C6/(SEQ ID NO: 13)
For *BMP3*
   5'-GTT TAA TTT TCG GTT TCG TCG TC-3'(SEQ ID NO: 14)
   5'-CTC CCG ACG TCG CTA CG-3'(SEQ ID NO: 15)
   5'-CGC CGA GGC GGT TTT TTG CG/3'C6/(SEQ ID NO: 16)
For *TFPI2*
   5'-TCG TTG GGT AAG GCG TTC-3'(SEQ ID NO: 17)
   5'-AAA CGA ACA CCC GAA CCG-3'(SEQ ID NO: 18)
   5'-GAC GCG GAG GCG GTT TTT TGT T/3'C6/(SEQ ID NO: 19)
The TFPI2 assay had a specific invasive oligo
   5'GCG GGA GGA GGT GCC-3'(SEQ ID NO: 20)
The primers and probe for detecting bisulfite-treated *ACTB* (β-actin reference gene) were
   5'-TTT GTT TTT TTG ATT AGG TGT TTA AGA-3'(SEQ ID NO: 21)
   5'-CAC CAA CCT CAT AAC CTT ATC-3'(SEQ ID NO: 22)
   5'-CCA CGG ACG ATA GTG TTG TGG/3'C6/(SEQ ID NO: 23)

Each plate included bisulfite-treated DNA samples, standard curve samples, positive and negative controls, and water blanks. Standard curves were made using 300 to 1000 target sequences cut from engineered plasmids. Bisulfite-treated sheared CpGenome™ universal methylated DNA (Zymo Research) and human genomic DNA (ATCC, Manassas, VA) were used as positive and negative controls. DNA strand number was determined by comparing a predetermined crossing threshold of the target gene to the standard curve of that assay. Percent methylation for each marker was determined by dividing the strand number of the methylated gene by the ACTB strand number and multiplied by 100.

### KRAS Mutations

The *KRAS* gene was first PCR amplified with primers flanking codons 12 and 13 using 10 µl of captured *KRAS* DNA as template. PCR was conducted with 0.025 U/µl hot start GoTaq® and 200 nM each primer in a 100 µl reaction volume at 95°C for 3 min, and then 15 cycles at 95°C 20 sec, 62°C 30 sec, and 72°C 30 sec. Primer sequences were
5'-AGG CCT GCT GAA AAT GAC TG-3'(SEQ ID NO: 24)
5'-CTA TTG TTG GAT CAT ATT CG TC-3'(SEQ ID NO: 25)

Each amplified sample was diluted 500-fold in nuclease-free water. Then, 10 µl of the 500-fold sample dilutions was added to 96-well PCR plate with an automated liquid handler (epMotion, Eppendorf, Hauppauge NY). QuARTS assays were then used to evaluate seven mutations at codons 12 and 13 of the *KRAS* gene. Mutation-specific forward primers and probes were
*KRAS* G12S mutation
   5'-CTT GTG GTA GTT GGA GCA A-3'(SEQ ID NO: 26)
   5'-GCG CGT CCA GTG GCG TAG GC/3'C6/(SEQ ID NO: 27)
*KRAS* G12C mutation
   5'-AAA CTT GTG GTA GTT GGA CCT T-3'(SEQ ID NO: 28)
   5'-GCG CGT CCT GTG GCG TAG GC/3'C6/(SEQ ID NO: 29)
*KRAS* G12R mutation
   5'-TAT AAA CTT GTG GTA GTT GGA CCT C-3'(SEQ ID NO: 30)
   5'-GCG CGT CCC GTG GCG TAG GC/3'C6/(SEQ ID NO: 31)
*KRAS* G12D mutation
   5'-ACT TGT GGT AGT TGG AGC TCA-3'(SEQ ID NO: 32)
   5'-GCG CGT CCA TGG CGT AGG CA/3'C6/(SEQ ID NO: 33)
*KRAS* G12V mutation
   5'-ACT TGT GGT AGT TGG AGC TCT-3'(SEQ ID NO: 34)
   5'-GCG CGT CCT TGG CGT AGG CA/3'C6/(SEQ ID NO: 35)
*KRAS* G12A mutation
   5'-AAC TTG TGG TAG TTG GAG ATG C-3'(SEQ ID NO: 36)
   5'- GCG CGT CCC TGG CGT AGG CA/3'C6/(SEQ ID NO: 37)
*KRAS* G13D mutation
   5'-GGT AGT TGG AGC TGG TCA-3'(SEQ ID NO: 38)
   5'-GCG CGT CCA CGT AGG CAA GA/3'C6/(SEQ ID NO: 39)

The reverse primer for each was
5'-CTA TTG TTG GAT CAT ATT CGT C-3'(SEQ ID NO: 40)

Each mutation assay was designed as a singleplex assay. QuARTS cycling conditions and reagent concentrations for *KRAS* were the same as those in the methylation assays. Each plate contained standards made of engineered plasmids, positive and negative controls, and water blanks, and was run in a LightCycler 480 (Roche). DNA strand number was determined by comparing a predetermined crossing threshold of the target gene to the standard curve for that assay. The concentration of each *KRAS* mutation marker in 50 µl was calculated based on the 500-fold dilution factor and an amplification efficiency of 1.95. This value was divided by the *ACTB* concentration in the methylation assay, and then multiplied by 100 to determine the percent mutation.

### Plasma Septin 9 Test (SEPT9)

Samples were prepared as follows: whole blood was centrifuged at 3200 rpm/1823 × g for 10 minutes; plasma was then re-spun at 3200 rpm/1823 × g for 10 minutes and aliquoted into 2 ml cryovials and frozen at -80°C. The time from venipuncture to freezing was less than 4 hours for all samples, and none had been previously thawed. Frozen samples (2 vials or approximately 4 ml per patient) were sent to ARUP Laboratories for SEPT9 testing using their commercially available assay (see, e.g., Heichman KA, et al. "Use of Septin 9 methylated DNA biomarker to detect cancer in the blood of colorectal cancer patients", Presented at ASCO-NCI-EORTC meeting in Philadelphia, October, 2010). The SEPT9 result was judged as positive if at least 1 out of 3 replicates exceeded a pre-defined marker level. These criteria for positivity yielded a specificity of 89%.

### Hemoglobin Assay

To quantify hemoglobin in stool using, *e.g.,* the HemoQuant test, the test is performed on one or more, preferably at least two buffered stool aliquots (each normalized to for the amount of stool, *e.g.,* 16 mg) per patient. Unlike immunochemical or guaiac type fecal blood tests, the HemoQuant test assays heme-derived porphyrin and is not affected by stool storage.

### Reference Method

Colonoscopy, in conjunction with histopathology on all colorectal lesions, served as the reference standard. In cases with multiple lesions, patients were classified based on their most advanced or largest neoplasm.

### Statistical analysis for sDNA data

A logistical regression model was developed using data from a training set to define a linear combination of age and stool marker variables that optimized the discrimination between case patients with a colorectal neoplasm (CRC or adenoma greater than 1cm) and controls with normal colonoscopy. The modeling strategy consisted of fitting age, quadratic effect of age, beta-actin, fecal hemoglobin, and the sDNA methylation markers into a base model and adding quadratic and pair-wise interactions of these variables using forward variable selection with entry and removal from the model based on a p-value of 0.05. A cutoff for *KRAS* mutation, defined *a priori,* resulted in only one control patient being positive. Because this extremely high specificity created modeling difficulties, the effect of mutant KRAS was added after variable selection; presence of mutant *KRAS* was considered predictive of having a significant lesion. The 90th or 95th percentiles of the linear discriminant score within control patients (specificity) were used to define test positivity.

The linear discriminant score with corresponding cutoff value was then applied to the test set. Sensitivity and specificity with corresponding 95% confidence intervals were estimated for each training set and each test set separately and for combined sets. The area under the empirical ROC curve (AUC) was used to compare the accuracy of nested logistic models and investigate the added value of each sDNA methylation marker to the overall linear discriminant score using a paired methods approach. The Chi-square test was used to assess the association of test positivity with categorical clinical and lesion characteristics. The association of continuous lesion size with the linear discriminant score was evaluated using piece-wise linear regression (inflection point at 3 cm) and tests between lesion subtypes were based on the change in fit between nested models. Statistical significance was defined as p<0.05.

Test positivity for both sDNA and SEPT9 tests was defined prior to un-blinding of patient disease status and was based on respective historical specificity cut-off values. Observed sensitivity and specificity for each test was estimated with corresponding 95% confidence intervals based on the exact binomial distribution. Fischer's exact test for paired proportions was used to compare sensitivity between the two markers overall and within disease subgroups. Plasma samples from patients with normal colonoscopy were used as process controls and for estimating the specificity of SEPT9. Descriminant scores from the sDNA and SEPT9 tests were combined to provide a combined descriminator of colorectal neoplasia for subjects as described below.

### EXAMPLE 1

During the development of embodiments of the technology provided herein, the sDNA and SEPT9 tests were evaluated as discriminators of colorectal cancer. The sDNA and SEPT9 were first evaluated for sensitivity on cancer and adenoma samples separately. The data from paired stool and plasma samples was also merged and re-evaluated for sensitivity as a combined descriminator for colorectal neoplasia.

### Patient and Lesion Characteristics

The study included a total of 147 patients (52 cases, 49 plasma controls, and 46 stool controls). Cases with paired plasma and stool samples comprised 52 patients with advanced adenoma or CRC. Among the 22 adenomas, median size was 2.0 cm (range 1.0-5.4 cm) and 55% were located at or proximal to the splenic flexure. Among the 30 CRCs, median size was 4.3 cm (0.8-8.3 cm); 50% were proximal; and 7 (23%), 7 (23%), 8 (27%), and 8 (27%) were Stage I, II, III, and IV, respectively.

### Neoplasm Detection Rates of the separate sDNApanel and plasma SEPT9 tests

Considering all neoplasm cases (large adenomas and CRC) with paired specimens, sensitivity was 85% (95% CI 72-93%) by sDNA compared to 40% (27-56%) by plasma SEPT9, p=0.0001. Because the quantity of plasma received from the archive fell below the desired threshold of 3.6 ml in 8 cases, tests were also compared on only those with plasma volumes ≥ 3.6 ml. In these 44 cases with ≥ 3.6 ml of plasma available for analyses, observed detection rates were essentially the same as in the whole group; sensitivity was 84% (70-93%) by sDNA and 39% (34-55%) by SEPT9, p=0.0001.

For the detection of large adenomas, sensitivity was 82% (95% CI 60-95%) by sDNA and 14% (3-35%) by SEPT9, p=0.0001.

For the detection of CRC, sensitivity was 87% (95% CI 69-96%) by sDNA and 60% (41-77%) by SEPT9, p=0.046. Respective detection rates for stages I, II, III, and IV cancers were 86%, 86%, 100%, and 75% by sDNA and 57%, 57%, 38%, and 88% by SEPT9. Among combined stages I-III, sDNA detected 91% (95% CI 71-99%) and SEPT9 detected 50% (28-72%), p=0.013; detection rates for stage IV CRC did not differ significantly between tests, p=0.56. See Figure 1. Detection of proximal CRC was 92% (64-100%) by sDNA and 46% (19-75%) by SEPT9, p=0.034; and detection of distal CRC was 81% (54-96%) by sDNA and 69% (46-89%) by SEPT9, p=0.48.

Among the 46 control stools, 3 (7%) were false-positives yielding an observed specificity by sDNA of 93% (95% CI 84-98%). Among the 48 control plasma samples, 13 (27%) were false-positives yielding an observed specificity by SEPT9 of 73% (58-85%). After reviewing Tumor Registry data and medical records on all 48 plasma control patients, one patient was found with a remote personal history of CRC that had not been disqualified from the study and 4 patients were identified with non-gastrointestinal malignancies (including one with multiple myeloma, one with lymphoma, and two with prostate cancer) who presented after the date that stools had been collected and archived; four of these 5 control patients were from the false-positive subset and one from the true-negative subset. If these 5 patients were excluded, then the re-calculated specificity for SEPT9 was 79% (95% CI 64-90%).

### Neoplasm Detection Rates of the paired sDNApanel plus plasma SEPT9 tests

Data from the sDNA and the SEPT9 tests were combined into a single discriminator and evaluated as a diagnostic tool for colorectal neoplasia (e.g., CRC).

For cancer, the sensitivity of the sDNA test was 87% and for the plasma SEPT9 test the sensitivity was 60% (p<0.05). See Figure 1. The sensitivity of the combination was 100%: in particular, 100% of cases missed by the SEPT9 test were called positive by the sDNA test and 100% of cases missed by the sDNA test were called positive by the SEPT9 test. See Table 1, above, and Table 2.

**TABLE 2 - sDNA and SEPT9 combined panel for cancer**

| **Cancer** Sensitivity All Matched Samples with | | SEPT9 test | | Total |
|---|---|---|---|---|
| | | Negative | Positive | |
| sDNA test | Negative | 0 | 4 | 4 |
| | Positive | 12 | 14 | 26 |
| | | | | Sensitivity: 87% |
| | | | | 95% CI: (69%, 96%) |
| Total | | 12 | 18 | 30 |
| | | | Sensitivity: 60% | |
| | | | 95% CI: (41%, 77%) | |

| | | | | |
|---|---|---|---|---|
| 68% Discordance; 100% of cases missed by Sept9 were called positive with sDNA test; 100% of cases missed by Prototype I were called positive with Sept9; | | | | |

The estimated sensitivity for the sDNA test alone was 87%, compared to 60% with plasma Sept9 alone (p=0.0455).

For adenoma, the estimated sensitivity for the sDNA test was 83%, compared to 14% for the SEPT9 test (p=0.0001). Adding SEPT9 to the sDNA test for adenoma detection did not have a significantly better predictive value relative to the sDNA panel alone. In particular, 79% of cases missed by the SEPT9 test were called positive with the sDNA test and 0% of cases missed by the sDNA test were called positive with the SEPT9 test. See Table 3.

**TABLE 3 - sDNA and SEPT9 combined panel for adenoma**

| **Adenoma** Sensitivity All Matched Samples with | | SEPT9 test | | Total |
|---|---|---|---|---|
| | | Negative | Positive | |
| sDNA test | Negative | 4 | 0 | 4 |
| | Positive | 15 | 3 | 18 |
| | | | | Sensitivity: 82% |
| | | | | 95% CI: (60%, 95%) |
| Total | | 19 | 3 | 22 |
| | | | Sensitivity: 14% | |
| | | | 95% CI: (3%, 35%) | |

| | | | | |
|---|---|---|---|---|
| 68% Discordance; 79% of cases missed by Sept9 were called positive with sDNA test; 0% of cases missed by sDNA test were called positive with Sept9; | | | | |

The estimated sensitivity for sDNA was 83% compared to 14% with Sept9 (p=0.0001)

## Claims

1. A method of screening for a colorectal neoplasm in a subject, the method comprising:
a) providing paired samples from a subject, wherein said paired samples comprise
i) a stool sample, and
ii) a blood sample or a plasma sample;
b) assaying said stool sample to create a first data set, wherein said assaying comprises measuring the methylation state of at least one DNA marker;
c) assaying said blood or a plasma sample to create a second data set, wherein said assaying comprises measuring the methylation state of at least one DNA marker;
d) combining said first data set and said second data set to form a paired data set;
e) analyzing said paired data set to determine the presence or absence of a methylation profile indicative of colorectal neoplasm in said subject.

2. The method of claim 1, wherein said assaying said stool sample comprises measuring the methylation states of a plurality of DNA markers, preferably at least three DNA markers, more preferably at least four DNA markers.

3. The method of claim 1 or claim 2, wherein said assaying said plasma sample comprises measuring the methylation state of at least one DNA marker not assayed in said stool sample.

4. The method of any one of claims 1-3, wherein said assaying said stool sample comprises measuring the methylation state of a marker selected from the group consisting of a *vimentin* gene, an *NDRG4* gene, a *BMP3* gene, and a *TFPI2* gene.

5. The method of any one of claims 1-3, wherein said assaying said stool sample comprises measuring the methylation states of a *vimentin* gene, an *NDRG4* gene, a *BMP3* gene, and a *TFPI2* gene.

6. The method of any one of claims 1-5, wherein said assaying of said plasma sample comprises measuring the methylation state of a *Septin 9* gene.

7. The method of any one of claims 1-6, wherein said assaying said stool sample further comprises detecting a mutant *KRAS* gene.

8. The method of any one of claims 1-7, wherein said assaying said stool sample further comprises detecting fecal hemoglobin.

9. The method of claim 1 wherein the assaying said stool sample and/or said blood or plasma sample comprises the use of a quantitative, allele-specific, real-time target and signal amplification assay.

10. The method of any one of claims 1-9, wherein the neoplasm comprises a neoplasm selected from the group consisting of a cancer, an adenoma, and a pre-cancerous neoplasm.

11. A method of screening for a colorectal neoplasm in a subject, the method comprising:
a) providing paired samples from a subject, wherein said paired samples comprise
i) a stool sample, and
ii) a blood sample or a plasma sample;
b) isolating a first DNA from said stool sample;
c) isolating a second DNA from said stool sample;
d) isolating a third DNA from said blood sample or from said plasma sample;
e) measuring a first methylation state of a first gene using the first DNA;
f) measuring a second methylation state of a second gene using the second DNA;
g) measuring a third methylation state of a third gene using the third DNA.
h) combining data from said measuring of said first methylation state, said second methylation state and said third methylation state to form a paired data set;
i) analyzing said paired data set to determine the presence or absence of a methylation profile indicative of colorectal neoplasm in said subject.

12. The method of claim 11, wherein said first and said second gene are selected from the group consisting of *vimentin* gene, an *NDRG4* gene, a *BMP3* gene, and a *TFPI2* gene and/or wherein said third gene is a *Septin 9* gene.

13. The method of claim 11 or claim 12, wherein isolating said first DNA from said stool sample and isolating said second DNA from said stool sample are performed sequentially.

14. The method of any one of claims 1-13, wherein said subject is human.

## Patentansprüche

1. Verfahren zum Screening auf eine colorektale Neoplasie bei einem Subjekt, wobei das Verfahren umfasst:
a) die Bereitstellung von gepaarten Proben von einem Subjekt, wobei besagte gepaarte Proben umfassen
i) eine Stuhlprobe und
ii) eine Blut- oder Plasmaprobe;
b) die Untersuchung der Stuhlprobe in einem Assay um einen ersten Datensatz zu erzeugen, wobei besagte Untersuchung die Messung des Methylierungszustandes von mindestens einem DNS-Marker umfasst;
c) die Untersuchung der besagten Blut- oder Plasmaprobe um einen zweiten Datensatz zu erzeugen, wobei besagte Untersuchung die Messung des Methylierungszustandes von mindestens einem DNS-Marker umfasst;
d) die Kombination des besagten ersten Datensatzes mit dem besagten zweiten Datensatz um einen gepaarten Datensatz zu bilden;
e) die Analyse des besagten gepaarten Datensatzes, um die An- oder Abwesenheit eines Methylierungsprofils zu bestimmen, das für eine colorektale Neoplasie in besagtem Subjekt indikativ ist.

2. Verfahren nach Anspruch 1, wobei besagte Untersuchung besagter Stuhlprobe in einem Assay die Messung der Methylierungszustände einer Vielzahl von DNS-Markern, vorzugsweise von mindestens drei DNS-Markern, weiter bevorzugt von mindestens vier DNS-Markern, umfasst.

3. Verfahren nach Anspruch 1 oder Anspruch 2, wobei besagte Untersuchung besagter Plasmaprobe in einem Assay die Messung des Methylierungszustandes von mindestens einem DNS-Marker umfasst, der nicht in dem Assay der Stuhlprobe untersucht wurde.

4. Verfahren nach einem der Ansprüche 1-3, wobei besagte Untersuchung besagter Stuhlprobe in einem Assay die Messung des Methylierungszustandes eines Markers umfasst, der ausgewählt ist aus der Gruppe bestehend aus einem *vimentin*-Gen, einem *NDRG4*-Gen, einem *BMP3*-Gen und einem *TFPI2*-Gen.

5. Verfahren nach einem der Ansprüche 1-3, wobei besagte Untersuchung besagter Stuhlprobe in einem Assay die Messung der Methylierungszustände eines *vimentin*-Gens, eines *NDRG4*-Gens, a *BMP3-Gens und eines TFPI2*-Gens umfasst.

6. Verfahren nach einem der Ansprüche 1-5, wobei besagte Untersuchung besagter Plasmaprobe in einem Assay die Messung des Methylierungszustandes eines *Septin 9*-Gens umfasst.

7. Verfahren nach einem der Ansprüche 1-6, wobei besagte Untersuchung besagter Stuhlprobe in einem Assay des Weiteren die Detektion eines mutierten *KRAS*-Gens umfasst.

8. Verfahren nach einem der Ansprüche 1-7, wobei besagte Untersuchung besagter Stuhlprobe in einem Assay des Weiteren die Detektion von fäkalem Hämoglobin umfasst.

9. Verfahren nach Anspruch 1, wobei die Untersuchung besagter Stuhlprobe und/oder besagter Blut- oder Plasmaprobe in einem Assay die Verwendung eines quantitativen, Allel-spezifischen, Echtzeit-Ziel- und Signalverstärkungsassay umfasst.

10. Verfahren nach einem der Ansprüche 1-9, wobei das Neoplasma ein Neoplasma umfasst, das ausgewählt ist aus der Gruppe bestehend aus einem Krebs, einem Adenom und einer präkanzerösen Neoplasie.

11. Verfahren zum Screening auf eine colorektale Neoplasie bei einem Subjekt, wobei das Verfahren umfasst:
a) die Bereitstellung von gepaarten Proben von einem Subjekt, wobei die besagten gepaarten Proben umfassen
i) eine Stuhlprobe und
ii) eine Blut- oder Plasmaprobe;
b) die Isolierung einer ersten DNS aus besagter Stuhlprobe;
c) die Isolierung einer zweiten DNS aus besagter Stuhlprobe;
d) die Isolierung einer dritten DNS aus besagter Blut- oder Plasmaprobe;
e) die Messung eines ersten Methylierungszustandes eines ersten Gens unter Verwendung der ersten DNS;
f) die Messung eines zweiten Methylierungszustandes eines zweiten Gens unter Verwendung der zweiten DNS;
g) die Messung eines dritten Methylierungszustandes eines dritten Gens unter Verwendung der dritten DNS;
h) die Kombination von Daten aus der Messung des besagten ersten Methylierungszustandes, des besagten zweiten Methylierungszustandes und des besagten dritten Methylierungszustandes, um einen gepaarten Datensatz zu bilden;
i) die Analyse des besagten gepaarten Datensatzes, um die An- oder Abwesenheit eines für colorektale Neoplasie in besagtem Subjekt indikativen Methylierungsprofils festzustellen.

12. Verfahren nach Anspruch 11, wobei besagtes erstes und besagtes zweites Gen ausgewählt sind aus der Gruppe bestehend aus dem *vimentin-Gen,* einem *NDRG4*-Gen, einem *BMP3-Gen* und einem *TFPI2*-Gen und/oder wobei besagtes drittes Gen ein *Septin 9*-Gen ist.

13. Verfahren nach Anspruch 11 oder Anspruch 12, wobei die Isolierung besagter erster DNS aus besagter Stuhlprobe und die Isolierung besagter zweiter DNS aus besagter Stuhlprobe nacheinander durchgeführt werden.

14. Verfahren nach einem der Ansprüche 1-13, wobei besagtes Subjekt ein Mensch ist.

## Revendications

1. Procédé de dépistage d'une néoplasie colo-rectale chez un sujet, le procédé comprenant :
a) la fourniture d'échantillons appariés d'un sujet, lesdits échantillons appariés comprenant
i) un échantillon de selles, et
ii) un échantillon de sang ou un échantillon de plasma ;
b) le dosage dudit échantillon de selles pour générer un premier ensemble de données, ledit dosage comprenant la mesure de l'état de méthylation d'au moins un ADN marqueur ;
c) le dosage dudit échantillon de sang ou d'un échantillon de plasma pour générer un deuxième ensemble de données, ledit dosage comprenant la mesure de l'état de méthylation d'au moins un ADN marqueur ;
d) la combinaison dudit première ensemble de données et dudit deuxième ensemble de données pour former un ensemble de données appariées ;
e) l'analyse dudit ensemble de données appariées pour déterminer la présence ou l'absence d'un profil de méthylation indicatif d'une néoplasie colo-rectale chez ledit sujet.

2. Procédé de la revendication 1, dans lequel ledit dosage dudit échantillon de selles comprend la mesure des états de méthylation d'une pluralité d'ADN marqueurs, de préférence au moins trois ADN marqueurs, plus préférablement au moins quatre ADN marqueurs.

3. Procédé de la revendication 1 ou la revendication 2, dans lequel ledit dosage dudit échantillon de plasma comprend la mesure de l'état de méthylation d'au moins un ADN marqueur non dosé dans ledit échantillon de selles.

4. Procédé de l'une quelconque des revendications 1 à 3, dans lequel ledit dosage dudit échantillon de selles comprend la mesure de l'état de méthylation d'un marqueur choisi dans le groupe constitué d'un gène de *vimentine,* un gène *NDRG4,* un gène *BMP3* et un gène *TFPI2.*

5. Procédé de l'une quelconque des revendications 1 à 3, dans lequel ledit dosage dudit échantillon de selles comprend la mesure des états de méthylation d'un gène de *vimentine,* un gène *NDRG4,* un gène *BMP3* et un gène *TFPI2.*

6. Procédé de l'une quelconque des revendications 1 à 5, dans lequel ledit dosage dudit échantillon de plasma comprend la mesure de l'état de méthylation d'un gène de *septine 9.*

7. Procédé de l'une quelconque des revendications 1 à 6, dans lequel ledit dosage dudit échantillon de selles comprend en outre la détection d'un gène *KRAS* mutant.

8. Procédé de l'une quelconque des revendications 1 à 7, dans lequel ledit dosage dudit échantillon de selles comprend en outre la détection d'hémoglobine fécale.

9. Procédé de la revendication 1 dans lequel le dosage dudit échantillon de selles et/ou dudit échantillon de sang ou de plasma comprend l'utilisation d'un dosage par amplification de cible et de signal en temps réel, allèle-spécifique, quantitative.

10. Procédé de l'une quelconque des revendications 1 à 9, dans lequel la néoplasie comprend une néoplasie choisie dans le groupe constitué d'un cancer, un adénome et une néoplasie précancéreuse.

11. Procédé de dépistage d'une néoplasie colo-rectale chez un sujet, le procédé comprenant :
a) la fourniture d'échantillons appariés d'un sujet, lesdits échantillons appariés comprenant
i) un échantillon de selles, et
ii) un échantillon de sang ou un échantillon de plasma ;
b) l'isolement d'un premier ADN à partir dudit échantillon de selles ;
c) l'isolement d'un deuxième ADN à partir dudit échantillon de selles ;
d) l'isolement d'un troisième ADN à partir dudit échantillon de sang ou dudit échantillon de plasma ;
e) la mesure d'un premier état de méthylation d'un premier gène au moyen du premier ADN ;
f) la mesure d'un deuxième état de méthylation d'un deuxième gène au moyen du deuxième ADN ;
g) la mesure d'un troisième état de méthylation d'un troisième gène au moyen du troisième ADN ;
h) la combinaison des données de ladite mesure dudit premier état de méthylation, dudit deuxième état de méthylation et dudit troisième état de méthylation pour former un ensemble de données appariées ;
i) l'analyse dudit ensemble de données appariées pour déterminer la présence ou l'absence d'un profil de méthylation indicatif d'une néoplasie colo-rectale chez ledit sujet.

12. Procédé de la revendication 11, dans lequel lesdits premier et deuxième gènes sont choisis dans le groupe constitué d'un gène de *vimentine,* un gène *NDRG4,* un gène *BMP3* et un gène *TFPI2* et/ou dans lequel ledit troisième gène est un gène de *septine 9.*

13. Procédé de la revendication 11 ou la revendication 12, dans lequel l'isolement dudit premier ADN dudit échantillon de selles et l'isolement dudit deuxième ADN à partir dudit échantillon de selles sont effectués séquentiellement.

14. Procédé de l'une quelconque des revendications 1 à 13, dans lequel ledit sujet est humain.
